(19) Europäisches Patentamt / European Patent Office / Office européen des brevets

(11) **EP 4 480 497 A1**

(12) **EUROPEAN PATENT APPLICATION**

(43) Date of publication:
**25.12.2024 Bulletin 2024/52**

(21) Application number: **23180717.3**

(22) Date of filing: **21.06.2023**

(51) International Patent Classification (IPC):
**A61K 47/62** (2017.01)  **A61K 47/64** (2017.01)
**A61K 47/68** (2017.01)  **A61P 35/00** (2006.01)

(52) Cooperative Patent Classification (CPC):
**A61K 47/6815; A61K 47/62; A61K 47/64;
A61K 47/6801; A61K 47/6887; A61K 47/6889;
A61K 51/10; A61K 51/1036; A61K 51/1093**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **RDP Pharma AG
8590 Romanshorn (CH)**

(72) Inventor: **The designation of the inventor has not yet been filed**

(74) Representative: **SONN Patentanwälte GmbH & Co KG
Riemergasse 14
1010 Wien (AT)**

Remarks:
The complete document including Reference Table(s) and the Sequence Listing(s) can be downloaded from the EPO website

(54) **BIO-CONJUGATES FOR ONCOLOGY**

(57)     The invention discloses a compound comprising or consisting of
(i) a polypeptide with the general formula (I):

$$X_0GX_1X_2GX_3X_4X_5GX_6X_7X_8GX_9X_{10}X_{11}X_{12}X_{13}X_{14},$$

wherein
G is glycine;
$X_0$ is present or not and, if present, is an amino acid linker;
$X_1$ is N or S, wherein N is asparagine and S is serine;
$X_2$ is S or T, wherein S is serine and T is threonine;
$X_3$ is present or not and, if present, is S, wherein S is serine;
$X_4$ is present or not and, if present, is G, wherein G is glycine;
$X_5$ is a basic polypeptide stretch consisting of 5 basic amino acid residues selected from R and K, wherein R is arginine and K is lysine, preferably wherein $X_5$ comprises at least 3 K amino acid residues, especially wherein $X_5$ is KKKKK or KRKKK;
$X_6$ is a basic amino acid residue selected from R and K, wherein R is arginine and K is lysine, preferably wherein $X_6$ is K;
$X_7$ is S or L, wherein S is serine and L is leucine;
$X_8$ is present or not and, if present, is a polypeptide stretch consisting of the amino acid sequence GLGS, wherein G is glycine, L is leucine and S is serine;
$X_9$ is a basic polypeptide stretch consisting of 3 basic amino acid residues selected from R and K, wherein R is arginine and K is lysine, preferably wherein $X_9$ comprises at least 2 K amino acid residues, especially wherein $X_9$ is KKK or KKR;
$X_{10}$ is present or not and, if present, is L or a polypeptide stretch consisting of the amino acid sequence DPL or DPC, wherein L is leucine, D is aspartic acid, P is proline, and C is cysteine;
$X_{11}$ is present or not and, if present, is a polypeptide stretch consisting of the amino acid sequence LR or GSGL, wherein L is leucine, R is arginine, G is glycine, and S is serine;
$X_{12}$ is present or not and, if present, is a basic polypeptide stretch with at least 20 % basic amino acid residues selected from K and R and at least a P residue, preferably wherein $X_{12}$ is selected from KYKPKL, KYKPKLGT, or $GX_3X_4X_5GX_6X_7X_8GX_9X_{10}$, wherein K, R, P, L, G, T, $X_3$, $X_4$, $X_5$, $GX_6$, $X_7$, $X_8$, $GX_9$, and $X_{10}$, are defined as above and wherein Y is tyrosine;
$X_{13}$ is present or not and, if present, is a polypeptide stretch consisting of the amino acid sequences GS, GST, GST, GSG, GSTG, or GSGL, wherein G, S, T and L are defined as above;
$X_{14}$ is present or not and, if present, is an amino acid linker; wherein the polypeptide has a length from 20 to 75 amino acid

**(Cont. next page)**

EP 4 480 497 A1

residues, preferably from 24 to 65 amino acid residues, especially from 25 to 60 amino acid residues; and

(ii) at least one payload molecule to be delivered into a biological cell,
wherein (i) and (ii) are covalently linked to each other.

**Description**

The present invention relates to a non-antibody-drug conjugate, a nuclease drug conjugate (NDC), having an antitumour drug conjugated via a linker structure moiety to a site-specific fusion protein of an endonuclease covalently bound to a tumour cell selective cell penetrating peptide, the conjugate being useful as an antitumour drug.

Antibody-drug conjugates (ADC) are molecules that enable the target-specific use of a high-potent cytotoxic drugs, bringing about a significant improvement of the therapeutic window for their therapeutic applications. ADCs consist of tumour targeting mAbs conjugated to cytotoxic payloads through a carefully designed chemical linker, enabling an advantageous mechanism of activity (MOA) of a concomitant selective targeting principle and highly potent effectiveness. The conjugation of a small molecule toxin to a hydrophilic antibody, can ameliorate the uptake of a hydrophobic cytotoxic payload, further upgrading the therapeutic index. It is the scope of this invention to provide functional bi-activity molecules that go far beyond the mAb - toxin carrier concept of an ADC. Today's molecular oncology research has led to a highly advanced knowledge of the genes and pathways involved in tumour induction, progression and maintenance suggesting hundreds of new molecular targets for therapeutic intervention. Consequently, many new drugs have been designed for oncology based on molecular targeting principles. The first generation of these drugs have been used in the clinics for over two decades. These drugs have greatly influenced modern pharmacology approaches and are now employed as new cancer therapies. Some of these drugs have increased survival rates in combination therapies used in second- or third-line regimens, however, the big breakthrough in first line treatments for most of the indications, and for solid tumours, are lagging initial expectations for effective molecules with mild side effects. In going through treatment options for most of the tumour indications combination regimens are applied and the normal life aggravating chemotherapeutics are still necessary for efficacy in many of the regimens. Due to their robustness, chemotherapeutics and radiotherapy are still the principal treatment options, mainly applied in chemotherapeutic regimens or prominently in combination regiments with targeted therapies and/or immunotherapies for most late-stage tumours. The insufficient tumour selective mode of action of cytotoxic drugs leads to narrow pharmaceutical windows, as frequently rapidly dividing healthy cells are attacked as well as cancer cells, leading to the side effects commonly associated with chemotherapy such as hair loss, sickness, cardiotoxicity, and tiredness. Likewise, contrary to expected mild toxicology profiles, most of the new targeted drug candidates show substantial side effects and the typically cytostatic efficacy profiles often result in a rather unsatisfying length of time to progression. In addition, multi-drug resistance is frequently observed. More recent immunotherapies, using CTL4, PD-1/PD-L1 Checkpoint Inhibitors in Tumour Immunotherapy targeting CTL4-A, PD1 or PDL1 receptors with suitable antibodies such as ipilimumab, tremelimumab, nivolumab, pembrolizumab and atezolizumab, were successfully introduced, but also these very promising reagents need combinations with chemotherapeutics for efficient therapeutic success. ADCs and NDCs have a fair chance to substitute for the robustness giving chemotherapeutic compound in many therapeutic regimens as well as for maintenance therapies.

Antibody therapies to surface antigens that are differential expressed in tumour cells versus somatic cells in homeostasis, can well successfully constitute novel therapeutic options. The mayor part of these biomolecules exhibit receptor signalling modifying modes of activity, enhanced by Antibody-dependent cellular cytotoxicity (ADCC) effects with superior safety profiles but lack of robustness and can cause on-target effects in healthy cells. Again, antibody containing therapeutic regimens frequently demand combinations with cytotoxic chemotherapeutics.

Along these lines, because of less than satisfactory results from first generation molecular targeting reagents in the clinics, physicians have suggested both a personalized medicine approach based on drug efficacy profiles and the treatment of cancer as a chronic disease, aiming to keep the tumour burden constantly low by changing regimens, as used for viral infections such as HIV. At the same time, a growing number in the art suggest the development of new, less toxic drugs that interfere with those pathways targeted by chemotherapeutic interventions resulting in robust cytotoxic effects such as metabolic pathways, DNA replication, DNA repair and cell division. These approaches will only have a chance if quality of life during therapy is not substantially altered, therefore only drugs with mild toxicity and a convenient pharmacokinetic profile will make the difference. NDC full fill these features. Antibody-drug conjugates (ADCs) have emerged as a promising class of new therapeutics that only in part, fulfil these requirements. ADCs are structurally comprised of an antibody conjugated to cytotoxic payloads by specially designed linkers. In most of the cases the cytotoxic compound comprises the active principle and the antibody contributes mainly to a differential, selective recognition of target receptors, ideally enriched in or unique to a given therapeutic target indication. In rare cases the antibodies by themselves exhibit target specific synergistic effects.

Despite the intense development of ADCs, the development of these complex molecules faces still many, only partially resolved, technical barriers related to the drug like properties of complex molecules, including plasma stability, payload dissociation, low blood retention time, non-sufficient tumour selectivity and thus on-target toxicity, minimal tumour penetration, decreased payload efficiency, immunogenicity, off-target toxicity, and drug resistance.

To develop this further ADC were successfully optimised. But still there is space for improvements, mainly slow and low cell penetrating efficiencies of antibodies and consequently the need for high toxin loads which in turn leads to stronger exposure of toxin during the serum circulation and from lysed cells. The antibodies show single type of receptor recognition, although efficient feature, often limits applications due to mutated or receptor negative populations and the presence, albeit to a lower extend of receptors also in healthy cells can induce unwanted on-target as well as off target events.

NDC the molecule of the invention addresses these vulnerabilities. A) low affinity onco-fetal receptors highly differential present in most tumour cells, are active also in common ADC receptor negative tumour cells such as double or triple negative breast tumours or metastatic linages. B) The presence of two, synergistic MOA principles in one NDE molecule presented allows for lower toxin load, thus arriving at concentrations which are not rate limiting, even for the worst case, if a substantial amount of the presented toxin would be accidental released in serum or non-intended compartments of an individual.

The use of small DDM peptides that cross cellular membranes, also called CPPs or protein transduction domains (PTDs), enables cellular drug delivery of endonucleases and can enhance drug delivery, reaching steady state levels equilibrium inside of the cells and in targeted compartments of tumour cells within 30 minutes to one hour. This is much faster than the uptake of the antibody targeted ADCs which show very much slower uptake kinetics of days and thus longer circulation in the body fluids with potential exposure of toxic payloads. Another object of the present invention is to provide CPPs which have the ability to efficiently translocate a biological membrane and provide tumour cell-type selective and/or intracellular compartment selective delivery of molecules, and thus have utility in therapy and/or as research tools.

Therefore, as CPP also called PTD or DDM, the present invention uses a compound comprising or consisting of

(i) a polypeptide with the general formula (I):

$$X_0GX_1X_2GX_3X_4X_5GX_6X_7X_8GX_9X_{10}X_{11}X_{12}X_{13}X_{14},$$

wherein

G is glycine;

$X_0$ is present or not and, if present, is an amino acid linker;

$X_1$ is N or S, wherein N is asparagine and S is serine;

$X_2$ is S or T, wherein S is serine and T is threonine;

$X_3$ is present or not and, if present, is S, wherein S is serine;

$X_4$ is present or not and, if present, is G, wherein G is glycine;

$X_5$ is a basic polypeptide stretch consisting of 5 basic amino acid residues selected from R and K, wherein R is arginine and K is lysine, preferably wherein $X_5$ comprises at least 3 K amino acid residues, especially wherein $X_5$ is KKKKK or KRKKK;

$X_6$ is a basic amino acid residue selected from R and K, wherein R is arginine and K is lysine, preferably wherein $X_6$ is K;

$X_7$ is S or L, wherein S is serine and L is leucine;

$X_8$ is present or not and, if present, is a polypeptide stretch consisting of the amino acid sequence GLGS, wherein G is glycine, L is leucine and S is serine;

$X_9$ is a basic polypeptide stretch consisting of 3 basic amino acid residues selected from R and K, wherein R is arginine and K is lysine, preferably wherein $X_9$ comprises at least 2 K amino acid residues, especially wherein $X_9$ is KKK or KKR;

$X_{10}$ is present or not and, if present, is L or a polypeptide stretch consisting of the amino acid sequence DPL or DPC, wherein L is leucine, D is aspartic acid, P is proline, and C is cysteine;

$X_{11}$ is present or not and, if present, is a polypeptide stretch consisting of the amino acid sequence LR or GSGL, wherein L is leucine, R is arginine, G is glycine, and S is serine;

$X_{12}$ is present or not and, if present, is a basic polypeptide stretch with at least 20 % basic amino acid residues selected from K and R and at least a P residue, preferably wherein $X_{12}$ is selected from KYKPKL, KYKPKLGT, or $GX_3X_4X_5GX_6X_7X_8GX_9X_{10}$, wherein K, R, P, L, G, T, $X_3$, $X_4$, $X_5$, $GX_6$, $X_7$, $X_8$, $GX_9$, and $X_{10}$, are defined as above and wherein Y is tyrosine;

$X_{13}$ is present or not and, if present, is a polypeptide stretch consisting of the amino acid sequences GS, GST, GST, GSG, GSTG, or GSGL, wherein G, S, T and L are defined as above;

$X_{14}$ is present or not and, if present, is an amino acid linker;

wherein the polypeptide has a length from 20 to 75 amino acid residues, preferably from 24 to 65 amino acid residues, especially from 25 to 60 amino acid residues; and

(ii) a payload molecule to be delivered into a biological cell,

wherein (i) and (ii) are covalently linked to each other.

The compound of the present invention comprises of a new class of CPPs which have an unexpected cell penetrating and transmembrane transport properties which allow efficient transport of nuclease payload macromolecules (often also referred to as "nuclease", "protein", "endonuclease molecule", "biologic", "polypeptide" etc.). The DDM-CPPs (often also referred to as "Drug Delivery Module (DDP)", "cell penetrating peptide (CPP)", "Protein Transduction Domain (PTD)", etc.) according to the present invention have also improved properties for addressing tumour cells, especially when they are covalently linked to a molecule effective in killing tumour cells. The suitability to use the CPPs according to the present invention for treating tumour patients resides in the efficient cell-penetrating function of the polypeptides which in turn are covalently fused to a functional tumour-toxin, thus containing two active principles in one covalently fused multimeric molecule that enhances the cytotoxic or cytostatic activity of the nuclease. This ideally brings about a highly efficient molecule active inside a tumour cell in a synergistic fashion. Accordingly, for the present invention preferably DDM sequences are chosen which are disclosed in PCT/EP2022/086215. Moreover, WO 2004/092194 A2 teaches the fusion of a single chain PvuII restriction endonuclease to a cell-penetrating peptide. Also the use of this construct together with her DDM sequences of the present invention is a preferred embodiment of the present invention.

Nuclease Drug Conjugates (NDC)

The scope of the invention are non-antibody drug conjugates consisting of a covalently coupled, recombinant fusion protein of an endonuclease with a tumour selective cell penetrating peptide covalently coupled to a linker that conjugates said fusion protein with a toxic payload exhibiting an antitumour activity and exhibit concomitant two anti-tumour activities in a single chain molecule. The molecules of the invention have the following formula where all the compounds are coupled covalently resulting in a single molecule, The order of the individual compounds is arbitrary.

**Nuclease-DDM-LINKER-Toxin**

**Nuclease** is a recombinant endonuclease which is, or is not modified by a homopolymer.

**DDM** is a tumour selective cell penetrating peptide (CPP)
**LINKER** is an extracellular stable chemical linker and intracellularly either stable, unspecific cleavable or selective cleavable. The linker covalently links a TOXIN to any residue of the nuclease molecule, multiple linkers can link multiple toxins to the same nuclease molecule.

**TOXIN** is a cytotoxic or cytostatic small molecule.

Within the NDC, subject to the invention, endonucleases that are enabled by DDM-CPP sequences to selectively target tumour cells and to penetrate tumour cell walls by energy dependent endocytosis shuttle cytotoxin-payloads, safely and differential in a systemic context to its place of action in a tumour cell. Due to the endogenous nuclease activity the NDC hydrolyses site specific non mutagenic DNA double-strand breaks leading eventually to a synergistic MOA with the conjugated toxin and allows for high efficacy and a broad pharmacological window. This is even further enhanced by the fact that NDC can bring together the two most robust principles of chemotherapy in one drug, DNA break and tubulin distortion in a target specific fashion.

The compounds provided with the present invention with the improved CPP moieties exhibit better efficiency during the membrane crossing and intracellular sorting processes. The CPPs according to the present invention are specifically suited as a platform to transport different payloads for different pharmaceutical applications. Moreover, the efficient CPPs according to the present invention also exhibit improved solubility in recombinant expression systems and show robust stability in production processes. This subsequently allows transfer and up-scale into API manufacturing processes of CPP-conjugates as compounds according to the present invention, bringing about pharmaceutical acceptable stability of a respective drug product.

According to a preferred embodiment, the CPP moiety in the compound according to the present invention is a compound with the general formula (I), wherein $X_1$ is N, $X_2$ is S, $X_3$ is S, $X_4$ is G, $X_5$ is KKKKK or KRKKK, $X_6$ is K, $X_9$ is KKK or KKR, and $X_{10}$ is L or a polypeptide stretch consisting of the amino acid sequence DPL or DPC, wherein N, S, G, R, L, D, P and C are defined as above.

According to a preferred embodiment, the number of arginine or cysteine residues in the CPP moiety with the general formula (I). Preferably, the compound according to the present invention has a general formula (I) which contains a single arginine and/or a single cysteine or not more than two arginine residues and/or not more than two cysteine residues. According to a specifically preferred embodiment, the CPP has a general formula (I) which lacks an arginine and/or cysteine residue, especially wherein the general formula (I) lacks both, arginine and cysteine residues.

The replacement of arginine residues and/or the exchange of arginine residues with lysine residues has shown to improve the stability of the compounds according to the present invention, both by preventing protease degradation in vivo (i.e., when the compounds according to the present invention are applied to human patients) and by preventing degradation in the course of the production process. Exchange of arginine by another amino acid, especially by lysine may e.g., prevent a protease site.

This lack of cysteine residues in the CPP moiety is, of course, independent of the use of cysteine residues in linking moieties which links the CPP to a endonuclease molecule (see below). Moreover, cysteine residues may also be used as preferred points of PEGylation to provide PEGylated compounds and enable also thiol toxin-payload molecule conjugation (see below).

According to another preferred embodiment, the nuclease compound according to the present invention comprises at least two polypeptides with the general formula (I), preferably wherein the CPP consists of two polypeptides with the general formula (I).

A specifically preferred embodiment of the present invention is a compound wherein the CPP is selected from the group GNSGSGKKKKKGKGSGLGSGKKKDPL (DDM34) and GNSGSGKKKKKGKGSGLGSGKKKDPLGSGLGSGK KKKKGKGSGLGSGKKKDPLGST (DDM36).

Preferred specific examples of the CPPs according to the present invention are GNSGSGKRKKKGKGLGKKRDPCLRKYKPKLGTGSTG(HHHHHH) (DDM11), GNSGKRKKKGKGSGLGSGKKRDPCLRKYKPKLGTGSTG(HHHHHH) (DDM18), GNSGSGKRKKKGKGLGKKRDPCLRKYKPKLGTGSTG(HHHHHH) (DDM21), GNSGSGKRKKKGKGSGLGSGKKRDPLGSTG(HHHHHH) (DDM26), GNSG(HHHHHH) GSTGGKRKKKGKGSGLGSGKKRDPL (DDM27), GNSGSGKRKKKGKGSGLGSGKKKDPLGSTG(HHHHHH) (DDM28), GNSG(HHHHHH)GSTGGKRKKKGKGSGLGSGKKKDPL (DDM29), GNSGSGKKKKKGKGSGLGSGKKRDPLGSTG(HHHHHH) (DDM30), GNSG(HHHHHH) GSTGGKKKKKGKGSGLGSGKKRDPL (DDM31), GNSGSGKKKKKGKGSGLGSGKKKDPLGSTG(HHHHHH) (DDM32), GNSG(HHHHHH)GSTGGKKKKKGKGSGLGSGKKKDPL (DDM33), GNSGSGKKKKKGKGSGLGSGKKKDPL (DDM34), GNSGSGKKKKKGKGSGLGSGKKKLGSTG(HHHHHH) (DDM35), GNSGSGKKKKKGKGSGLGSGKKKDPLGSGLGSGKKKKKGKGSGLGSGKKKDPLGST (DDM36), and

    GNSGSGKKKKKGKGSGLGSGKKKDPLGSGLGSGKKKKKGKGSGLGSGKKKDPLGSTG(HHHHHH)
    GST (DDM44),

wherein (HHHHHH; a histidine tag) may be present or not; preferably GNSGSGKRKKKGKGLGKKRDPCLRKYKPKLGTGSTG (DDM11), GNSGKRKKKGKGSGLGSGKKRDPCLRKYKPKLGSTG (DDM18), GNSGSGKRKKKGKGLGKKRDPCLRKYKPKLGTGSTG (DDM21), GNSGSGKRKKKGKGSGLGSGKKRDPLGSTG (DDM26), GNSGGSTGGKRKKKGKGSGLGSGKKRDPL (DDM27), GNSGSGKRKKKGKGSGLGSGKKKDPLGSTG (DDM28), GNSGGSTGGKRKKKGKGSGLGSGKKKDPL (DDM29), GNSGSGKKKKKGKGSGLGSGKKRDPLGSTG (DDM30), GNSGGSTGGKKKKKGKGSGLGSGKKRDPL (DDM31), GNSGSGKKKKKGKGSGLGSGKKKDPLGSTG (DDM32), GNSGSGKKKKKGKGSGLGSGKKKDPL (DDM34), GNSGSGKKKKKGKGSGLGSGKKKDPLGSGLGSGK KKKKGKGSGLGSGKKKDPLGST (DDM36), and GNSGSGKKKKKGKGSGLGSGKKKDPLGSGLGSGKKKKKGKGS GLGSGKKKDPLGSTGGST (DDM44), especially GNSGSGKKKKKGKGSGLGSGKKKDPLGSGLGSGKKKKKGKGS GLGSGKKKDPLGST (DDM36), and GNSGSGKKKKKGKGSGLGSGKKKDPLGSGLGSGKKKKKGKGSGLGSGKKK DPLGSTGGST (DDM44).

According to a preferred embodiment, the polypeptide with the general formula (I) in the compound according to the present invention is covalently coupled to the C-terminus of an endonuclease molecule to be delivered into a tumour cell, preferably a compound selected from the group

    (CROMOC01)

    SHPDLNKLLELWPHIQEYQDLALKHGINDIFQDNGGKLLQVLLITGLTVLPGREGNDAVDNAGQE
    YELKSINIDLTKGFSTHHHMNPVIIAKYRQVPWIFAIYRGIAIEAIYRLEPKD-
    LEFYYDKWERKWYSDGHKDINNPKIPVKYVMEHGTKIYGNSGSGKKKKKGKGSGLGSGKKKDPLG
    SGLGSGKKKKKGKGSGLGSGKKKDPLGST,

    (CROMOC02)

    SHPDLNKLLELWPHIQEYQDLALKHGINDIFQDNGGKLLQVLLITGLTVLPGREGNDAVDNAGQE
    YELASINIDLTKGFSTHHHMNPVIIAKYRQVPWIFAIYRGIAIEAIYRLEPKD-
    LEFYYDKWERKWYSDGHKDINNPKIPVKYVMEHGTKIYGNSGSGKKKKKGKGSGLGSGKKKDPLG
    SGLGSGKKKKKGKGSGLGSGKKKDPLGST,

(CROMOC36)

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGGSGGSSHPDLNKLLELWPHIQEY
QDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

(CROMOC60)

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGGSGGSSHPDLNKLLELWPHIQEY

QDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

(CROMOC62)

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCSGGSSHPDLNKLLELWPHIQEY
QDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

(CROMOC63)

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCSGGSSHPDLNKLLELWPHIQE
YQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

(CROMOC70)

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCSGGSSHPDLNKLLELWPHIQEY
QDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

(CROMOC87)

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCSGGSSHPDLNKLLELWPHIQE
YQDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

(CROMOC88)

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCCSGGSSHPDLNKLLELWPHIQ
EYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST, and

(CROMOC89)

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCCSGGSSHPDLNKLLELWPHIQ
EYQDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

(CROMOC90)

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIY**GSGGCAACSGGS**SHPDLNKLLELWPHI
QEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST**,**

(CROMOC91)

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIY**GSGCAACAACSGS**SHPDLNKLLELWPH
IQEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST**.**

The term "coupled" or "covalently coupled" preferably relates to a covalent peptidic coupling, e.g., the coupling of two moieties of the compound according to the present invention via peptidic linkage thereby forming a polypeptide. Of course, other coupling (e.g. via S-S bonding or other chemical (covalent) coupling) may also be used to link the different moieties in the present compound; however, peptide bonds obtained in recombinant fusion proteins are the preferred way to (covalently) couple the moieties of the DDM peptide with the general formula (I) and the endonuclease molecule (with or without a linker, etc.) with each other to form a polypeptide with a single amino acid chain.

According to another preferred embodiment, the DDM polypeptide with the general formula (I) is covalently coupled to the C-terminus of a payload molecule to be delivered into a biological cell which is a class II restriction endonuclease, preferably PvuII or a subunit thereof, wherein the endonuclease molecule is optionally attached at its C-terminus to a linker molecule comprising 8 to 12 amino acids comprising or consisting of glycine, serine and/or cysteine residues, and wherein the polypeptide with the general formula (I) is covalently coupled to the C-terminus or the N-terminus of the endonuclease molecule or the linker molecule, wherein the compound comprises one or two endonuclease molecule monomer(s), optionally separated by the linker molecule comprising 8 to 12 amino acids comprising or consisting of glycine, serine and/or cysteine residues.

Preferably, the polypeptide with the general formula (I) is covalently coupled to the C-terminus of an endonuclease molecule to be delivered into a biological cell, wherein the compound comprises a first endonuclease molecule which is the first subunit of PvuII linked at its C-terminus to a linker molecule comprising 8 to 12 amino acids comprising or consisting of glycine, serine and/or cysteine residues, wherein the linker molecule is attached at its C-terminus to the second subunit of PvuII and wherein the polypeptide with the general formula (I) is covalently coupled to the C-terminus of the second subunit of PvuII. An example of such a compound according to the present invention may have the following structure (unless stated otherwise: always from N- to C-terminus): PvuII-1st subunit-DDM. Another example is a compound having the following structure: PvuII-1st subunit - 8-12 amino acid linker comprising glycine, Serine and Cysteine residues - PvuII-2nd subunit-DDM.

The PvuII restriction endonuclease is 157 amino acid residues long and has naturally a homodimeric form. The homodimer can easily be converted into a single polypeptide chain (sc PvuII). The two subunits may be tandemly linked through a short peptide linker (see above). The arrangement of a single-chain PvuII (sc PvuII) may be (2-157)-linker-(2-157), where (2-157) represents the amino acid residues of the enzyme subunit. PvuII endonuclease activity as sc enzyme may be expressed at high level as a soluble protein. The purified enzyme was shown to have the molecular mass expected for the designed sc protein. The cleavage specificity of the sc PvuII is indistinguishable from that of the wild-type (wt) enzyme.

These preferred CPP moieties according to the present invention (the "Drug Delivery Module" or "DDM" sequences) turned out to significantly to increase membrane crossing and desired intracellular sorting activities, increase stability towards proteolytic degradation, increase solubility of a given payload (especially a polypeptide payload) or the expression of a polypeptide encoded by a polynucleotide molecule (as payload) and simplify payload purification.

According to a preferred embodiment, the compound according to the present invention additionally comprise a payload molecule to be delivered into a biological cell covalently attached to the polypeptide with the general formula (I), preferably a chemotherapeutic molecule, a cytotoxic molecule, a DNA damaging molecule, an anti-metabolite molecule, a therapeutic molecule, a small molecule with therapeutic effect inside a biological cell, a DNA molecule, an RNA molecule, an antibody molecule or an antibody derivative having an antibody-like function, a restriction endonuclease, a nicking enzyme, or DNA- or RNA-dependent endonucleases which show double strand breaking nuclease double strand/single strand breaking activity or no nuclease activity, more preferred wherein the payload molecule is a class II restriction endonuclease, such as PvuII, EcoRV, PvuII, Hinfl, or a sc homodimer, a subunit or a functional fragment thereof; especially wherein the payload molecule is selected from the group microtubule inhibitors, especially auristatin, maytansinoids, tubulysins; cytotoxic small molecules with a molecular mass of 300 to 1300 Daltons, Dimethyl-SGD-1882, CAS1222490-34-7, SN-38, 7-Ethyl-10-hydroxycamptothecin, CAS86639-52-3, Duocarmycin, CAS118292-34-5, Tubulysin-beta, Cas 1943604-24-7, Tubulysin A(TubA) CAS 205304-86-5, Tubulysin, Tubulysin B, M, IM1, IM2, IM3, CAS205304-86-5, Tubulysin C, Tubulysin D, Tubulysin E, Tubulysin F, Tubulysin G, Tubulysin H, Tubulysin I, Maytansinoid AP-3 CAS 66547-09-Auristatin E CAS 160800-57-7, Auristatin F CAS163768-50-1, Dolastatin 10 CAS10417-88-4, Maytansinol CAS57103-68-1, Monomethyl auristatin D (MMAD) CAS203849-91-6, Monomethylauristatin F(MMAF) CAS745017-94-1, Monomethylauristatin E (MMAE) CAS474645-27-7, DM1 Maytansinoid CAS139504-50-0, DM4 Maytansinoid CAS799840-96-3, Maytansine DM3 CAS 796073-54-6, Pyrrolobenzodiazepine (PBDs), Rebeccamycin Sandramycin CAS100940-65-6,10-Deacetyl-7-xylosyl paclitaxel CAS90332-63-1, alpha-Amanitin CAS23109-05-9, gamma-Amanitin, Ansamitocin P-3 CAS66584-72-3, Calicheamicin CAS108212-75-5,

(CP01)

```
SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIY,
```

(CP02)

```
SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIY,
```

(CP36)

```
SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGGSGGSSHPDLNKLLELWPHIQEY
QDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIY,
```

(CP60)

```
SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGGSGGSSHPDLNKLLELWPHIQEY
QDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIY,
```

(CP62)

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCSGGSSHPDLNKLLELWPHIQEY
QDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIY,

(CP63)

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCSGGSSHPDLNKLLELWPHIQE
YQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIY,

(CP70)

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCSGGSSHPDLNKLLELWPHIQEY
QDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIY,

(CP87)

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCSGGSSHPDLNKLLELWPHIQE
YQDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIY,

(CP88)

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCCSGGSSHPDLNKLLELWPHIQ
EYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIY,

(CP89)

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCCSGGSSHPDLNKLLELWPHIQ
EYQDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIY,

(CP90)

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCAACSGGSSHPDLNKLLELWPHI
QEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST, and

(CP91)

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGCAACAACSGSSHPDLNKLLELWPH
IQEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST.

In a specifically preferred embodiment of the present invention, the compound according to the present invention comprises or consists of at least two payload molecule (ii), preferably a single chain restriction enzyme as a first (ii) and a toxin as second (ii) covalently coupled to (i), preferably in the order (ii) single chain restriction enzyme - (i) - (ii) toxin, wherein the toxin is preferably selected from microtubule inhibitors, especially auristatin, maytansinoids, tubulysins; cytotoxic small molecules with a molecular mass of 300 to 1300 Daltons, Dimethyl-SGD-1882, CAS1222490-34-7, SN-38, 7-Ethyl-10-hydroxycamptothecin, CAS86639-52-3, Duocarmycin, CAS118292-34-5, Tubulysin-beta, Cas 1943604-24-7, Tubulysin A(TubA) CAS 205304-86-5, Tubulysin, Tubulysin B, M, IM1, IM2, IM3, CAS205304-86-5, Tubulysin C, Tubulysin D, Tubulysin E, Tubulysin F, Tubulysin G, Tubulysin H, Tubulysin I, Maytansinoid AP-3 CAS 66547-09-Auristatin E CAS 160800-57-7, Auristatin F CAS163768-50-1, Dolastatin 10 CAS10417-88-4, Maytansinol CAS57103-68-1, Monomethyl auristatin D (MMAD) CAS203849-91-6, Monomethylauristatin F(MMAF) CAS745017-94-1, Monomethylauristatin E (MMAE) CAS474645-27-7, DM1 Maytansinoid CAS139504-50-0, DM4 Maytansinoid CAS799840-96-3, Maytansine DM3 CAS 796073-54-6, Pyrrolobenzodiazepine (PBDs), Rebeccamycin Sandramycin CAS100940-65-6,10-Deacetyl-7-xylosyl paclitaxel CAS90332-63-1, alpha-Amanitin CAS23109-05-9, gamma-Amanitin, Ansamitocin P-3 CAS66584-72-3, and Calicheamicin CAS108212-75-5.

Site specific endonucleases - CROMOC

The CROMOC class of molecules represents a new type of anticancer drug with a novel mechanism of action. They are highly stable enzymatic molecules composed of two monomeric endonuclease subunits covalently connected by selected linker sequences. CROMOC molecules are of synthetic or from procaryotic origin and for immunomodulation and immunoreaction-protection are shielded by covalently coupled long chain poly-ethylene-glycol residues. Contained within these biomolecules are peptides which allow to target CROMOC for entry into tumour cells. This targeting principle allows for a highly enriched preference targeting for primary as well as metastatic malignancies. CROMOC compounds are capable to enter tumour cells due to a covalent attachment of tumour selective cell penetrating peptides (CPP) called drug delivery molecules (DDM) . CROMOC molecules penetrate the cell wall in an ATP dependent fashion for most of the tumour cells by clathrin dependent endocytosis and/or by caveolin dependent endocytosis. Following cell import, CROMOC compounds are sorted into the nucleus of a tumour cell where they bind to specific cognate sites on DNA and exhibit their DNA double strand hydrolysing endo-nucleolytic function for chromatin DNA as well as extrachromosomal DNA copies in a site-specific fashion. Depending on the quality of the endonuclease in each CROMOC molecule, a corresponding DNA cognate site can be present as a single DNA locus up to hundreds or thousands of DNA cuts per cell. CROMOC molecules with single strand nicking activities or no-nuclease activities can be obtained as well. The integrated endonucleases introduce DNA double-strand breaks in the loosely packed chromatin which is characteristic of highly proliferating cells. This process results in neoplastic growth inhibition and eventually apoptosis of tumour cells.

The profile of the robust mechanism of action (MOA) of CROMOC resembles most closely radiotherapy and the class of chemotherapeutics that target rapidly dividing cancer cells with a MOA which directly or indirectly induces DNA damage. Among these agents, DNA alkylators and crosslinkers cyclophosphamide, chlorambucil, melphalan. Other examples of DNA-alkylating agents used in cancer treatment include nitrosoureas e.g., carmustine, lomustine, bendamustine, semustine and triazenes, e.g., dacarbazine and temozolomide. Likewise alkylating-like platinum agents' cisplatin, carboplatin, oxaliplatin, picoplatin. Antimetabolic inhibitors such as methotrexate the primary antifolate used in chemotherapy, aminopterin, and newer antifolates, such as pemetrexed, inhibit the dihydro-folate reductase enzyme to block the synthesis of nucleotides. Another antifolate, ralitrexed, directly inhibits thymidylate synthase. The pyrimidine analogs 5-fluorouracil (5-FU), capecitabine, floxuridine, and gemcitabine, and the purine analogs 6-mercaptopurine, 8-azaguanine, fludarabine, and cladribine whose incorporation of purine and pyrimidine analogues into DNA during the S phase causes DNA replication to fail. Topoisomerase I antagonists such as camptothecin lead to single strand breaks, and terminal to Topoisomerase II interacting inhibitors are DNA double strand breaks. Anthracyclines such as doxorubicin, daunorubicin, oxorubicin, idarubicin, eporubicin, nemorubicin all Topoisomerase II inhibitors most closely resemble very similar activity profiles with CROMOCs. However, CROMOC molecules do not introduce mutagenic DNA breaks, free radicals, or cause cardiotoxic effects rate limiting for anthracyclines. and most advantageously, due to the fused DDM sequence, targeting of CROMOC is strongly enriched for malignant cells in a systemic context. CROMOC works at low nanomolar to sub-nanomolar concentrations whereas most of the chemotherapeutics work in the low $\mu$M range.

Site specific endonucleases can be chosen from eucaryotic nuclear and mitochondrial endonucleases and RNase H. In a specific embodiment, endonucleases are chosen from procaryotic restriction enzymes. Preferred restriction endonuclease are type II restriction endonuleases, especially selected from AatII, AbaSI, Acc65I, AccI, AciI, AclI, AcuI, AfeI, AflII, AflIII, AgeI, AhdI, AleI, AluI, AlwI, AlwNI, ApaI, ApaLI, ApeKI, ApoI, AscI, AseI, AsiSI, AvaI, AvaII, BaeGI, BaeI, BamHI, BanI, BanII, BbsI, BbvCI, BbvI, BccI, BceAI, Bcgl, BciVI, BclI, BcoDI, BfaI, BfuAI, BfuCI, BglI, BglII, BlpI, BmgBI, BmrI, BmtI, BpmI, Bpu10I, BpuEI, BsaAI, BsaBI, BsaHI, BsaI, BsaJI, BsaWI, BsaXI, BseRI, BseYI, Bsgl, BsiEI, BsiHKAI, BsiWI, BslI, BsmAI, BsmBI, BsmFI, BsmI, BsoBI, Bsp1286I, BspCNI, BspDI, BspEI, BspHI, BspMI, BspQI, BsrBI, BsrDI, BsrFI, BsrGI, BsrI, BssHII, BssKI, BssSαI, BstAPI, BstBI, BstEII, , Bst, BstUI, BstXI, BstYI, BstZ17I, Bsu36I, BtgI, BtgZI, BtsαI, BtsCI, Cac8I, ClaI, CspCI, CviAII, CviKI-1, CviQI, DdeI, DpnI, DpnII, DraI, DraIII, DrdI, EaeI, EagI, EarI, EciI, Eco53kI, EcoNI, EcoO109I, EcoP15I, EcoRI, EcoRV, FatI, FauI, Fnu4HI, FokI, FseI, FspEI, FspI, HaeIII, HgaI, HhaI, HincII, HindIII, HinfI, HinP1I, HpaI, HpaII, HphI, Hpy166II, Hpy188I, Hpy188III, Hpy99I, HpyAV, HpyCH4III, HpyCH4IV, HpyCH4V, I-CeuI, I-SceI, KasI, KpnI, LpnPI, MboI, MboII, MfeI, , MluCI, MluI, MlyI, MmeI, MnlI, MscI, MseI, MslI, MspA1I, MspI, MspJI, MwoI, NaeI, NarI, Nb.BbvCI, Nb.BsmI, Nb.BsrDI, Nb.BtsI, NciI, NcoI, NdeI, NgoMIV, NheI, NlaIII, NlaIV, NmeAIII, NotI, NruI, NsiI, NspI, Nt.AlwI, Nt.BbvCI, Nt.BsmAI, Nt.BspQI, Nt.BstNBI, Nt.CviPII, PacI, PaeR7I, PciI, PflFI, PflMI, PI-PspI, PI-SceI, PleI, PluTI, PmeI, PmlI, PpuMI, PshAI, PsiI, PspGI, PspOMI, PspXI, PstI, PvuI, PvuII, RsaI, RsrII, Sac, SacII, SalI, SapI, Sau3AI, Sau96I, SbfI, ScaI, ScaI, ScrFI, SexAI, SfaNI, SfcI, SfiI, SfoI, SgrAI, SmaI, SmlI, SnaBI, SpeI, SphI, SspI, StuI, StyD4I, StyI, SwaI, Taqαl, TfiI, TseI, Tsp45I, TspMI, TspRI, Tth111I, XbaI, XcmI, XhoI, XhoI , XmaI, XmnI, and ZraI; as well as any nicking variants thereof or versions thereof with no nuclease activity (Sequence-specific DNA nicking endonucleases. Shuang-yong Xu, Biomolecular Concepts, De Gruyter August 7, 2015).

CROMOC62, a recombinant protein, is the lead compound in this class. CROMOC62 has pan cancer activity and functions for most of the cancer cells assessed. It has been tested extensively using *in vitro* as well as *in vivo* preclinical models with different human or mouse tumour cell lines (including glioblastoma, pancreas cancer, neuroblastoma and lung, colon and ovarian carcinomas, hepatocellular cancer, T and B cell lymphoma, multiple myeloma, and xenograft, orthotopic tumour models and metastasis allograft models. CROMOC62 induces potent cytotoxic effects on human tumour cells, with $EC_{50}$ in the low nanomolar range. Changes in cell cycle progression are also seen. Various studies have demonstrated the antitumour efficacy for CROMOC molecules administered subcutaneously or intravenously on several human and murine tumour models with good tolerability at effective doses. It is such a type of endonuclease that can advantageously be adapted for NDC molecules. The highly efficient nuclease activity can substitute DNA metabolism interfering chemotherapeutics allowing for low toxicity profiles and a broader pharmacological window.

Drug delivery module DDM

CROMOC finds multiple onco-fetal cell surface receptors. Cell penetrating peptide-based tumour selective Drug Delivery Modules (DDM) fused to CROMOC molecules recognize multiple cell surface receptors, strongly enriched in primary and metastatic tumour cells.

According to a preferred embodiment, the compound according to the present invention additionally comprises a homopolymer moiety covalently attached to the polypeptide with the general formula (I). Although such homopolymer-containing CPP compounds are in principle identified in the prior art as being advantageous to enhance CPP function in a druggable context (s. e.g. WO 2020/214846 A1 and the further documents cited in the search report to this international application), this is challenged by the resulting size of the molecule due to modifications with polymers and endosome trafficking. Moreover, the use of polymer therapeutics was reported to be hindered, because the cell membrane barrier often impedes their intracellular delivery.

The CPP moieties in the compound according to the present invention turned out to be so powerful in delivering payload through the cell barriers, the addition of polymers, especially homopolymers, can be foreseen in the compounds of the present invention so that the advantages accepted in the present field for such polymers attached to CPPs, including the ability of a certain compound to be adjusted with respect to the pharmaco-kinetic (PK) and pharmaco-dynamic (PD) properties of a given payload, are present for the compounds according to the present invention without the drawbacks of the enlarged size, the low cell type selectivity and the overall equal tissue distribution. Preferably, the homopolymer of the compound according to the present invention is selected from the group of polyethylene glycol (PEG), especially a PEG with a MW of 5 to 30 kDa; dextran, polysialic acids, hyaluronic acid, dextrin, hydroxyethyl starch, poly(2-ethyl 2-oxazoline, etc. (Pasut, Polymers 6 (2014), 160-178; Grigoletto et al., Nanomed. Nanobiotechnol. 2020, e1689); alternatively, also polypeptide techniques, such as protein conjugates with XTEN peptides or PASylation are available.

All polymer therapeutics used in practically relevant therapeutic applications are water soluble polymers. One of the most commonly employed polymers in polymer therapeutics is PEG, also specifically preferred for the present invention. PEG is approved for human administration by various routes of administration, e.g., mouth, injection, or dermal application. The structure of linear PEG is $HO-(CH_2-CH_2-O)_n-H$, where n indicates the number of repeats of the ethylene oxide unit in the PEG. PEG is a linear or branched, neutral polyether, and is commercially available in a variety of molecular mass; the polymerization can be controlled such that the molecular mass distribution is narrow. PEG derivatives can have different sizes with typically molecular weights ranging from hundreds to thousands of Da. PEG molecules used for polymer molecules are preferentially highly purified molecules with little or no diol impurities. Preferred PEG moieties used for the invention are mono-disperse or show only narrow molecular mass range differences (+/- 5% or better +/- 3%). Many PEG derivatives are available on the market or can be produced with known methods in the art.

The present invention therefore preferably relates to a PEGylated compound. Many of the benefits of PEGylated therapeutics lie in the properties of PEGs. PEGs are neutral, hydrophilic polymers that are soluble in water and a variety of organic solvents. Further, PEGs are inert, non-toxic, have a low immunogenicity, and the polymer is easily cleared from the body, mainly through the kidney for molecules with a molecular mass below 20 kDa, or through a combination of kidney and liver for molecules with molecular mass above 20 kDa. Up today, the maximum PEG molecular mass used for the preparation of polymer therapeutics is 40 kDa. PEGylation plays an important role in the stabilization of drugs, reduction of their antigenicity and decrease in the drug doses, besides augmenting the biodistribution ability via binding biologics onto their surfaces. PEGs possess the most suitable quality for preparation of physiologically active drug and biologics. Various functional groups are available and enable the introduction of the PEG chains into drugs, enzymes, phospholipids and other biologics. Covalent conjugation of hydrophobic macromolecules with activated PEGs leads to the formation of macromolecular micelles, which allow homogeneous dispersion of hydrophobic drugs in aqueous media.

Coupling of PEG or PEG derivatives to polypeptides (such as the polypeptide with the general formula (I)) can be obtained by coupling of PEG-NHS derivatives to polypeptide amines (PEG-NHS + polypeptide-$NH_2$) such as epsilon groups of lysine, coupling of PEG-aldehyde derivatives to the $NH_2$ group of polypeptides (PEG-Ald + polypeptide-$NH_2$), including secondary amines such as the N-terminus of a polypeptide, frequently used aldehyde linker include methoxy-PEG-CO$(CH_2)$nCOO-NHS, whereas n represents an integer of 1 to 3; or coupling of PEG-maleimide derivatives to the SH-group of polypeptide (PEG-Maleimide + polypeptide-SH) such as coupling to exposed cysteines contained within a polypeptide or engineered to the polypeptide by recombinant techniques, or coupling of PEG-$NH_2$ derivatives to the COOH group of polypeptides (PEG-$NH_2$ + polypeptide-COOH) such as surface exposed glutamic acid of a polypeptide or coupling of PEG-p-nitrophenyloxycarbonyl derivatives to the $NH_2$ group of polypeptides (PEG-NP + polypeptide-$NH_2$).

PEG derivatives can be a mono-functional linear PEGs, such as NHS active esters/carbonate, p-nitrophenyl carbonate PEG aldehyde PEG, aminopropyl PEG, aminoethyl PEG, thiol PEG, maleimide PEG, aminoxy PEG, hydrazide PEG, iodoacetamide PEG; or a bi-functional PEG, such as NHS-PEG, amine PEG, thiol PEG, maleimide PEG, or a multi-arm PEGs such as a 4-arm-PEG or an 8-arm-PEG; or a branched PEG such as a 2-arm branched PEG, 3-arm branched PEG, 4-arm branched PEG or a lysine branched PEG; or a heterofunctional PEG, such as Boc-protected-amino-PEG-carboxylic acid, 9-fluorenylmethyloxycarbonyl-protected-amino-PEG-carboxylic acid, maleimide-PEG-carboxylic acid, maleimide-PEG-NHS ester/carbonate, amino-PEG-carboxylic acid, Boc-protected-amino PEG-NHS carbonate, protected-mercapto-PEG NHS ester, 9-flourenylmethyloxycarbonyl-protected-amino-PEG-NHS ester, azido-PEG-NHS ester/carbonate, azido-PEG-amine, Biotin-PEG-NHS ester/carbonate, Biotin-PEG-maleimide, Biotin-PEG-amine; or also a forked PEG such as modified as a NHS-PEG, amine PEG or a maleimide PEG. A variety of PEG derivatives has been developed for such applications. Most polymer-macromolecules are chemical synthesized and can be coupled using linker technology known in the art to virtually all the known classes of therapeutically suitable molecules such as nucleic acids, PNA, lipids, small molecules peptides or proteins or mixtures thereof. Most of these molecules and in particular molecules with an extending molecular mass of more than >1000 Da are at least in part synthesized with molecular recombinant methods and it would be of advantage to have a polymer-macromolecule at disposal that can be produced recombinant as well including those polymers that can be produced as covalent fusion allowing for single step expression. Overall, covalent conjugation of polymers, e.g. PEG, with small molecule drugs and/or biological macromolecules such as polypeptides or polynucleotides is a promising approach for pharmaceutical applications, since such conjugates display altered (improved) pharmacokinetic properties, including a longer *in vivo* half-life; reduced immunogenicity; reduced toxicity; protection against proteolysis; improved water solubility; and increased pH and thermal stability; while the biological activity of the small molecule drug and/or the biological macromolecule is commonly retained in those conjugates.

A specifically preferred embodiment of the present invention is a compound wherein the (single chain) nuclease moiety (as a first payload (ii)) is connected to the N-terminus of the polypeptide (i) (which is preferably a CROMOC molecule as explicitly disclosed and referred to herein), wherein a toxin (as a second payload molecule (ii) is connected to an amine group (or to a cysteine residue) of the polypeptide (i); wherein the moieties are preferably connected by linker molecules (especially cleavable linkers). This preferred embodiment is preferably PEGylated, especially at the N-terminus of the polypeptide (i) or at a cysteine residue.

The compounds according to the present invention do not show undesirable off target effects and do not require frequently high therapeutic dosing, as has been reported for prior art polymer-CPP compounds.

In fact, the compounds according to the present invention generally show sufficient and often advantageous target specificity in selecting target selective active principles. The compounds according to the invention help to increase localized tissue distribution and to obtain selective cell type specificity. Thus, the compounds according to the present invention provide better target specificity to therapeutics, especially also to polymeric therapeutics. Such polymeric therapeutics encompass polymer-macromolecule conjugates, drug-polymer conjugates, and supramolecular drug-delivery systems. Besides a favourable biodistribution, these polymer therapeutics can accomplish several further objectives to optimize for CPP activity and adapt the pharmacokinetics profile of a polymer.

The combination of the new CPP moieties provided with the present invention together with polymer-modified macromolecular single activity profile compounds, or CPPs together with polymer-modified macromolecular multiple activity profile compounds can be engineered to be sorted to specific locations (specific biodistribution) after systemic treatment in vivo. Moreover, such compounds (even with a polymeric moiety attached) show efficient cross membrane passing by receptor mediated energy dependent endocytosis pathways. For designing the compounds according to the present invention, it turned out to be advantageous (and often compulsory for effective transfer of many payload molecules) to not attach the CPP via a polymeric moiety to the payload molecule but to link the polymeric moiety and the payload molecule to different amino acid residues of the CPP moiety of the compound according to the present invention.

According to a preferred embodiment, the compound according to the present invention additionally comprises a restriction endonuclease as a payload molecule to be delivered into a biological cell covalently attached to the polypeptide with the general formula (I), preferably a class II restriction endonuclease, especially a PvuII restriction endonuclease or a derivative thereof wherein the derivative is a single chain PvuII restriction endonuclease, such as the derivative with the amino acid sequence

SHPDLNKLLELWPHIQEYQDLALKHGINDIFQDNGGKLLQVLLITGLTVLPGREGNDAVDNAGQE YELKSINIDLTKGFSTHHHMNPVIIAKYRQVPWIFAIYRGIAIEAIYRLEPKDL-EFYYDKWERKWYSDGHKDINNPKIPVKYVMEHGTKIY,

SHPDLNKLLELWPHIQEYQDLALKHGINDIFQDNGGKLLQVLLITGLTVLPGREGNDAVDNAGQE YELKSINIDLTKGFSTHHHMNPVIIAKYRQVPWIFAIYRGIAIEAIYRLEPKDLEFYYDKWERKW YSDGHKDINNPKIPVKYVMEHGTKIYGSGG,

SGGSSHPDLNKLLELWPHIQEYQDLALKHGINDIFQDNGGKLLQVLLITGLTVLPGREGNDAVDN
AGQEYELKSINIDLTKGFSTHHHMNPVIIAKYRQVPWIFAIYRGIAIEAIYRLEPKDLEFYYDKW
ERKWYSDGHKDINNPKIPVKYVMEHGTKIY,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSG,

MSHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKDLEFYYDKWERK
WYSDGHKDINNPKIPVKYVMEHGTKIY,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGSGGC,

CSGGSSHPDLNKLLELWPHIQEYQDLALKHGINDIFQDNGGKLLQVLLITGLTVLPGREGNDAVD
NAGQEYELKSINIDLTKGFSTHHHMNPVIIAKYRQVPWIFAIYRGIAIEAIYRLEPKDLEFYYDK
WERKWYSDGHKDINNPKIPVKYVMEHGTKIY,

MSHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKDLEFYYDKWERK
WYSDGHKDINNPKIPVKYVMEHGTKIYGSGGCSGGSSHPDLNKLLELWPHIQEYQDLALKHGIND
IFQDNGGKLLQVLLITGLTVLPGREGNDAVDNAGQEYELKSINIDLTKGFSTHHHMNPVIIAKYR
QVPWIFAIYRGIAIEAIYRLEPKDLEFYYDKWERKWYSDGHKDINNPKIPVKYVMEHGTKIY,

MSHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKDLEFYYDKWERK
WYSDGHKDINNPKIPVKYVMEHGTKIYGSGGSGGSSHPDLNKLLELWPHIQEYQDLALKHGINDI
FQDNGGKLLQVLLITGLTVLPGREGNDAVDNAGQEYELKSINIDLTKGFSTHHHMNPVIIAKYRQ
VPWIFAIYRGIAIEAIYRLEPKDLEFYYDKWERKWYSDGHKDINNPKIPVKYVMEHGTKIY,

MSHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKDLEFYYDKWERK
WYSDGHKDINNPKIPVKYVMEHGTKIYGSGGSGGS,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGSGGSGGS,

GSGGSGGSSHPDLNKLLELWPHIQEYQDLALKHGINDIFQDNGGKLLQVLLITGLTVLPGREGND
AVDNAGQEYELKSINIDLTKGFSTHHHMNPVIIAKYRQVPWIFAIYRGIAIEAIYRLEPKDLEFY
YDKWERKWYSDGHKDINNPKIPVKYVMEHGTKIY,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIY**GSGGCAACSGGS**SHPDLNKLLELWPHI
QEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

or

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIY**GSGCAACAACSGS**SHPDLNKLLELWPH
IQEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST.

Therefore, a specifically preferred embodiment of the compound of the present comprises as payload two monomeric subunits or parts thereof of a type II restriction endonuclease. Preferably, two monomeric alpha-helical subunits of a type II restriction endonuclease can be covalently connected by a linker. Further preferred, the two monomeric alpha-helical subunits can be subunits of PvuII. Specifically preferred is a compound comprising two monomeric subunits or parts thereof of the type II restriction endonuclease PvuII covalently connected by a linker to the CPP moiety of the compound of the present invention. The linker is preferably an amino acid linker of 1 to 20 amino acid residues in length, preferably from 1 to 15 amino acid residues, more preferred from 7 to 13 amino acid residues, especially of 8 to 12 amino acid residues. Preferred amino acid linkers are linkers comprising or consisting of a cysteine, serine or glycine residue (or two, three four or five consecutive cysteine or glycine residues), especially a single or two adjacent cysteine residue(s); or an amino acid linker comprising glycine and serine residues or comprising or consisting of the amino acid sequences GSG, SGG, GGC, GCS, CSG, GGS, GSGG, SGGS, GSGGC, CSGGS, GSGGSGGS, GSGGCCSGGS, GSGGCSGGS, GSGGCAACSGGS or GSGCAACAACSGS.

Type II restriction endonucleases are reviewed e.g. by Pingoud et al. (NAR 29 (2001), 3705-3727; and NAR 42 (2014), 7489-7527); the common structure and function of type II restriction endonucleases is therefore well available to the person skilled in the art. A "derivative" of a restriction endonuclease is a polypeptide which is modified compared to the wild-type restriction endonuclease but still comprises the main function of the wild-type restriction endonuclease, i.e. the specific cleavage property, i.e. the ability to cut a nucleic acid molecule at a sequence-specific site. For the preferred example of PvuII, this means that a PvuII derivative is a derivative of the wild-type PvuII polypeptide which is modified compared to the wt PvuII polypeptide but still comprises the function of wild-type PvuII to specifically recognising the double-stranded DNA sequence 5'-CAGCTG-3' and cleave after G-3 (Cheng et al., EMBO J. 13 (1994), 3927-3935).

According to a preferred embodiment, the polypeptide with the general formula (I) in the compound of the present invention is covalently linked to another moiety by a linker, wherein the another moiety is preferably a payload molecule to be delivered into a biological cell, a labelling group, and/or a homopolymer.

A preferred embodiment is a compound according to the present invention, wherein the compound further comprises a capping group, preferably an alkoxy, especially a methoxy, ethoxy, propoxy, or butoxy group; a halogen atom; or a tosylate; isocyanate, hydrazine hydrate, maleimide, orthopyridyl disulfide, N-succinimidyloxy, sulfo-N-succinimidyloxy, 1-benzotriazol, 1-imidazolyloxy, p-nitrophenyloxy, or aldehyde.

Another aspect of the present invention is drawn to the compound according to the present invention, for use in the treatment of a tumour patient, preferably wherein the tumour patient is suffering from neuroblastoma, colon carcinoma, hepatocellular carcinoma, Small Cell Lung carcinoma, ovarian carcinoma, lung carcinoma, bladder carcinoma, prostate carcinoma, uterus carcinoma, cervix carcinoma, placental carcinoma, breast carcinoma, kidney carcinoma, liver carcinoma, pancreas carcinoma, muscle carcinoma, skin carcinoma, connective tissue carcinoma, bone carcinoma, and any of these carcinomas wherein the patient has already developed metastases, especially for the treatment of a patient having neuroblastoma, colon carcinoma, hepatocellular (liver) carcinoma, Small Cell Lung carcinoma, lung carcinoma, breast carcinoma, pancreas carcinoma, and any of these carcinomas wherein the patient has already developed metastases.

Another aspect of the present invention is drawn to a method of treatment of a tumour patient wherein an effective amount of the compound according to the present invention is administered to a patient in need thereof, preferably wherein the tumour patient is suffering from neuroblastoma, colon carcinoma, hepatocellular carcinoma, Small Cell Lung carcinoma, ovarian carcinoma, lung carcinoma, bladder carcinoma, prostate carcinoma, uterus carcinoma, cervix carcinoma, placental carcinoma, breast carcinoma, kidney carcinoma, liver carcinoma, pancreas carcinoma, muscle carcinoma, skin carcinoma, connective tissue carcinoma, bone carcinoma, and any of these carcinomas wherein the patient has already developed metastases, especially for the treatment of a patient having neuroblastoma, colon carcinoma, hepatocellular (liver) carcinoma, Small Cell Lung carcinoma, lung carcinoma, breast carcinoma, pancreas carcinoma, and any of these carcinomas wherein the patient has already developed metastases.

Another aspect of the present invention is drawn to the use of a compound according to the present invention for the manufacture of a medicament, preferably for the treatment of a tumour patient, more preferred for the treatment of a patient having neuroblastoma, colon carcinoma, hepatocellular carcinoma, Small Cell Lung carcinoma, ovarian carcinoma, lung carcinoma, bladder carcinoma, prostate carcinoma, uterus carcinoma, cervix carcinoma, placental carcinoma, breast carcinoma, kidney carcinoma, liver carcinoma, pancreas carcinoma, muscle carcinoma, skin carcinoma, connective tissue carcinoma, bone carcinoma, and any of these carcinomas wherein the patient has already developed metastases, especially for the treatment of a patient having neuroblastoma, colon carcinoma, hepatocellular (liver) carcinoma, Small Cell Lung carcinoma, lung carcinoma, breast carcinoma, pancreas carcinoma, and any of these carcinomas wherein the patient has already developed metastases.

A "CPP" as used herein means an amino-acid sequence, or polynucleotide encoding the same, which facilitates active transport of a biological macro-molecule across a biological membrane or a physiological barrier. Transduction across a biological membrane or a physiological barrier can be determined by various processes, for example by a cell penetration test having a first incubation step for the PTD conjugated to a marker in the presence of culture cells, followed by a fixating step, and then detection of the presence of the marked peptide inside the cell. Preferably, the CPP activity of a given molecule is tested according to the model system disclosed in the example section using the CROMOC molecule as a payload model. Alternatively, detection can be accomplished with an incubation of the CPP in the presence of labelled antibodies and directed against the CPP, followed by detection in the cytoplasm or in immediate proximity of the cell nucleus, or even within it, of the immunologic reaction between the CPP's amino acid sequence and the labelled antibodies. Cell penetration tests are well known to those skilled in the art.

The term "capping group" as used herein means any suitable chemical group which, depending upon preference, is unreactive or reactive with other chemical moieties. Accordingly, the capping group is selected to provide monofunctionally, i.e. the terminal aldehyde group, or bifunctionality, i.e. an aldehyde group on one terminus and a different functional moiety on the opposite terminus. If the capping group is unreactive with other chemical moieties, then the structure of the resulting polymer aldehyde derivative is monofunctional and therefore can covalently bond with only one chemical moiety of interest. In other words, in the case that the capping group is unreactive, the terminal aldehyde group of the compound of the present invention permits ready covalent attachment to a chemical moiety of interest, for example, to the $\alpha$-amino group of a polypeptide. Suitable capping groups are generally known in the art, for example, those disclosed in WO 2004/013205. Suitable non reactive capping groups include, for example, alkoxy, e.g. methoxy, ethoxy, propoxy, or butoxy; halogen atom (i.e. fluorine, chlorine, bromine, or iodine atom); or tosylate; and suitable reactive capping groups include, for example, isocyanate, hydrazine hydrate, maleimide, orthopyridyl disulfide, N-succinimidyloxy, sulfo-N-succinimidyloxy, 1-benzotriazol, 1-imidazolyloxy, p-nitrophenyloxy, or aldehydes.

The terms "group", "functional group", "moiety", "active moiety", "active compound", "effective compound", "target", "target molecule", "radical", etc. are somewhat synonymous in the chemical arts and are used in the art and herein to refer to distinct, definable portions or units of the compound according to the present invention and to units that perform some function or activity and are reactive with other molecules or portions of molecules. In this sense a small molecule drug or a polypeptide residue can be considered a functional group or moiety when coupled to a compound of the present invention.

Small Molecule Cytotoxic-Payloads

Payloads, also known as "cytotoxic molecules" or "warheads", are important factors affecting the properties and activities of NDCs. Ideally, the payload-activity is selected that it causes synergistic cytotoxic or cytostatic effects together with the nuclease activity contained in the NDC for a given tumour indication. Cytotoxic small molecules have a preferential molecular mass of 300 to 1300 Daltons. The mechanisms of cytotoxic molecules and their synergistic activity determine efficacy and adverse reactions of NDCs. The commonly used cytotoxic, molecules which are frequently natural-product based including microtubule inhibitors (such as auristatin, maytansinoids, tubulysines), DNA damaging agents (such as calicheamicin, duocarmycins, anthracyclines, pyrrolobenzodiazepines (PBD) derivates) and DNA replication inhibitors (amatoxin and quinoline alkaloid (SN-38)). More detailed examples of payloads include but are not limited to the substance-classes derived from, are: Calicheamicin gamma-1, CAS108212-75-5 carbonyl)-7,8-dihydro-3H-pyrrolo [3,2-e]indole-6-carbonyl]-4-methoxy-7,8-dihydro-3H-pyrrolo[3,2-e]indole-6-carboxamide Dimethyl-SGD-1882, CAS1222490-34-7, SN-38, 7-Ethyl-10-hydroxycamptothecin, CAS86639-52-3, Duocarmycin, CAS118292-34-5, Tubulysin-beta, Cas 1943604-24-7, Auristatin E CAS 160800-57-7, Auristatin F CAS163768-50-1, Dolastatin 10 CAS10417-88-4, Maytansinol CAS57103-68-1, Monomethyl auristatin D (MMAD) CAS203849-91-6, Monomethylauristatin F(MMAF) CAS745017-94-1, Monomethylauristatin E (MMAE) CAS474645-27-7, DM1 Maytansinoid CAS139504-50-0, DM4 Maytansinoid CAS799840-96-3, Maytansine DM3 CAS 796073-54-6, Pyrrolobenzodiazepine (PBDs), Rebeccamycin Sandramycin CAS100940-65-6,10-Deacetyl-7-xylosyl paclitaxel CAS90332-63-1, alpha-Amanitin CAS23109-05-9, gamma-Amanitin, Ansamitocin P-3 CAS66584-72-3, Calicheamicin CAS108212-75-5, Daun02 CAS290304-24-4, Daunorubicin-HCl CAS 23541-50-6, Daunorubicin (RP13057) CAS20830-81-3, Doxorubicin(Adriamycin) CAS 23214-92-8, Doxorubicin-HCl(Adriamycin) CAS 25316-40-9, Methotrexate(WR19039; CL14377) CAS 21672, Monomethyl auristatin D HCl (MMAD HCl) CAS 173441-26-4, Monomethylauristatin F HCl(MMAF HCl) CAS 1415246-68-2, Paclitaxel CAS 33069-62-4, PF-06380101 CAS 1436391-86-4, Nemorubicin CAS 108852-90-0 , PNU-159682 CAS 202350-68-3, Taltobulin (HTI-286; SPA-110) CAS 228266-40-8, Taltobulin trifluoroacetate (HTI-286; SPA-110) CAS 228266-41-9, Triptolide CAS 38748-32-2, Tubulysin A(TubA) CAS 205304-86-5, Tubulysin, Tubulysin B, M, IM1, IM2, IM3, CAS205304-86-5, Tubulysin C, Tubulysin D, Tubulysin E, Tubulysin F, Tubulysin G, Tubulysin H, Tubulysin I, Maytansinoid AP-3 CAS 66547-09-9, Cryptophycin CAS 124689-65-2, Boc-Nme-Val-Val-Dil-Dap-OH CAS, Duocarmycin SA CAS 130288-24-3, 17-AEP-GA Actinomycin X2, Aeruginosin 865, Agrochelin A, Agrochelin B, 17-AH-Geldanamycin , Ansatrienin B, AZD4547, Aphidicolin, Apoptolidin, AZD8055, Bafilomycin A1, Chaetocin, Chaetoglobosin A, Chaetoglobosin, Cinerubin B CAS 35906-51-5, Colchicine CAS 64-86-8, Combretastatin-A4 CAS 117048-59-6, Compound CL0485 CAS 723340-57-6, Cordycepin CAS 73-03-0, Cucurbitacin B CAS 6199-67-3, Cucurbitacin E CAS 18444-66-1, Curvulin CAS 19054-27-4, Cyclopamine CAS 4449-51-8, Dolastatin 15 CAS 123884-00-4, Epothilone B CAS 152044-54-7, CP201S, CP201R, VS035, OC974, MP33, beta-Amanitin CAS 21150-22-1, 4-methyl-Ahtea-Puwainaphycin F, Ferulenol CAS 6805-34-1, Fumagillin CAS 23110-15-8, Geldanamycin CAS 30562-34-6, Glucopiericidin A CAS 108073-65-0, 17-GMB-APA-GA, Gramicidin A CAS 11029-61-1, Herboxidiene CAS 142861-00-5, HL-100-AL1-R01 (H-3137), 9-Hydroxyellipticine, hydrochloride CAS 52238-35-4, Hygrolidin CAS 83329-73-1, Hypothemycin CAS 76958-67-3, Ilimaquinone CAS 71678-03-0, Isatropolone A, Isofistularin-3 CAS 87099-50-1, Ixabepilone, CAS 219989-84-1, JW 55 CAS 664993-53-7, Lactacystin CAS 133343-34-7, Luisol A CAS 225110-59-8, Mechercharmycin A CAS 822520-96-7, Mensacarcin CAS 808750-39-2, Microcolin B, Microcystin LR CAS 169789-55-3, Muscotoxin A, Myoseverin CAS 267402-71-1, Mytoxin B CAS 105049-15-8, N-acetyl Calicheamicin g1 CAS 108212-76-6, Nocuolin A, Okilactomycin CAS 111367-04-5, Oligomycin B CAS 11050-94-5, Phallacidin CAS 26645-35-2, Phalloidin CAS 17466-45-4, Phytosphingosine CAS 554-62-1, Piericidin A CAS 2738-64-9, Pironetin CAS 151519-02-7, Podophyllotoxin CAS 518-28-5, Polyketomycin CAS 200625-47-4, Pseudolaric acid B CAS 82508-31-4, Pseurotin A CAS 58523-30-1, epsilon-Amanitin CAS 21705-02-2, Puwainaphycin F, Quinaldopeptin CAS 130743-07-6, Rebeccamycin CAS 93908-02-2, Ro 5-3335 CAS 30195-30-3, Safracin B CAS 87578-99-2, Sanguinarine CAS 2447-54-3, Sinefungin CAS58944-73-3, Telomestatin CAS 265114-54-3, Thiocolchicine CAS 2730-71-4, Tolytoxin, Tripolin A CAS 1148118-92-6, Tubastatin A HCl CAS 1310693-92-5, DOXO-EMCH CAS 151038-96-9,Rachelmycin CAS 69866-21-3, Seco-Duocarmycin SA CAS 144667-38-9, DGN462 CAS 1394079-41-4, T785, Hexynoyl meropenem, Sulfo-DGN462 (sodium) CAS 1401203-09-5, SG3199 CAS 1595275-71-0,

Linker

The linker connects the nuclease and the cytotoxic payload and is a key component in the function of NDCs. The linker imparts to NDC the necessary high stability in the circulation, and specific release of the payload inside the cells of the target tissue. In addition, release must conserve the localization and function of the nuclease inside the nucleus of a target cell as well, enabling a bifunctional MOA, requesting site directed nucleolytic activity and payload activity in the same cell. To achieve these requirements, state of the art linker designs from the ADC field, where a vast variation of specific purpose linkers is known, can be adapted for NDCs developments.

At the high level, suitable linker designs can be divided according to their cleavage method into two linker principles. Either implementation as a cleavable linker, where a chemical trigger in its structure can be efficiently cleaved to release the cytotoxic payload in the target cell, or a stable, non-cleavable linker style. In contrast to the cleavable linker, there are no chemical triggers in the later, and the linker is part of the payload and is processed during endocytosis.

Although ADCs have achieved great progress in adaptive linker design, future development is necessary to reduce, and bring to a minimum the unspecific release of payloads in healthy tissues, to limit off-target toxicity concomitant with a broader therapeutic window. Same principles apply also for linkers used in NDC design. One must consider, compared to ADCs, NDCs in general show a shorter circulation-retention in the serum with a circulation half-life of 24 hours this contrasts with most ADCs which on average show serum circulation for more than 14 days, however NDCs exhibit very fast uptake with a saturation within 30 minutes at active, low nanomolar concentrations of NDCs due to the DDM sequence. In contrast ADC uptake can take days. These points are advantageous for NDC and can be considered in designing novel linkers for NDC purposes. However, ADC linker research done in the art can adapted for NDC linkers accordingly. The general structure comprises the roles of the chemical trigger, a principle that enables cleavage of a linker at the desired compartment, the linker-nuclease-DDM attachment and the linker-payload attachment. Commonly used linkers include the following examples. More detailed examples of linkers include but are not limited to the examples below.

Non-cleavable linkers

Differing from cleavable linkers, non-cleavable linkers have no structural chemical trigger for payload release. Examples are succinimidyl 4-(N-maleimidomethyl) cyclohexane-1-1carboxylate (SMCC), 2-(maleimidomethyl)-1,3-dioxane (MD), pyrrolobenzodiazepine (PBD) dimers, PEG linkers containing alkynes or piperazine, PEG linkers with intermediates of alkyne, triazole and piperazine, Mal-PAB linker.

Cleavable linkers

Cathepsin cleavable triggers, Dipeptide trigger, valinecitrulline (Val-Cit) linker, cBu-Cit-containing linkers, triglycyl peptide trigger(CX), valine-alanine (Val-Ala) trigger, Acid-cleavable triggers, Hydrazone trigger, Carbonate trigger, Silyl ether trigger, Glutathione (GSH)-cleavable triggers, Disulfide trigger, Fe(II) cleavable trigger, 1,2,4-Trioxolane trigger, Glycosidase cleavable triggers, β-Glucuronide trigger, β-Galactoside trigger, Phosphatase cleavable triggers, pyrophosphate trigger, Sulfatase cleavable trigger, arylsulfate trigger,Photoresponsive cleavable triggers, Heptamethine cyanine fluorophore trigger, O-Nitrobenzyl trigger, PC4AP trigger, Bioorthogonal cleavable triggers, dsProc trigger.

Linker-endonuclease attachments of the linker

Linker-nuclease attachments act as a bridge connecting the linker and nuclease. Challenge is the heterogeneous DAR values resulting in mixtures of different DAR values. Site-specific conjugation technology and chemical structural modification of the linker-nuclease attachment. Site-specific conjugation through nuclease engineering in the case is achieved by introducing one or more cysteines in the molecule for conjugation as is the case for CROMOC62 and CROMOC 63, and the corresponding nuclease minus molecules CROMOC60 and CROMOC70 respectively as compared to CROMOC 36 that does not contain a cysteine. Maleimide attachment, thiolmaleimide, thiosuccinimide, N-phenyl maleimide attachment, Bis(vinylsulfonyl)piperazine attachment, Pt(II)-based attachment.

Linker-payload attachments of the linker

Linker payload attachment strongly depends on the accessible chemistry of the payload, attachment can lower the activity of the toxin which is taken into consideration when choosing a specific chemistry. Examples are but not limited to the following.

Carbamate attachment and carbonate attachment, quaternary ammonium attachment for connecting payloads with a tertiary amine, quaternary ammonium-based glucQ-AE linker , ortho-hydroxyprotected aryl sulfate (OHPAS) attachment for payloads containing an aromatic-OH based on highly stable diaryl sulfate structures.

METHODS for the coupling of toxins to the nuclease

Methods and strategies for a covalent conjugation of cytotoxic or cytostatic reagents to endonucleases-DDM.

Chemical Covalent Conjugation Methods

Covalent coupling methods analytics and purification of protein-conjugates with chemical molecules are well described in the art (Mero et al., Conjugation of Poly(Ethylene Glycol) to Proteins and Peptides: Strategies and Methods. In Methods in Molecular Biology; 2011; Vol. 751, pp 95-129. https://doi.org/10.1007/978-1-61779-151-2 8). The development of an efficient and successful conjugation method requires reliable analytical methods for linker-payload reagents and endonuclease-DDM-payload conjugates at various stages of the process. Linker-payload reagents contribute as a substantial part to the manufacturing costs of nuclease-conjugates, and their purity impacts the conjugation efficiency and the product quality. The determination of the exact molecular weight, determining protein concentration and drug to protein ratio (DPR), the presence of reactive and non-reactive impurities, the degree of activation and the putative presence of payload or nuclease homodimers, potentially present depending on the coupling method used, in the starting material and final product must be carefully evaluated. To evaluate the degree of activation and to detect the presence of impurities, payloads and linker-payloads can be evaluated by both $^1$H- and $^{13}$C-NMR analyses. This technique requires few milligrams of polymer and suitable deuterated solvents: dimethylsulfoxide (DMSO-d$_6$), chloroform (CDCl$_3$) or water (D$_2$O). Usually, CDCl$_3$ or DMSO are preferred for the analysis of those activated linker-payload reagents that can be hydrolyzed in D$_2$O.

The most used linker-payload molecules for protein modification is methoxy- where only one terminal end of the polymer can be activated while the other is capped with the methoxy group, preventing undesired intra or inter-molecular cross-linking.

During the anion polymerization of ethylene oxide initiated by CH$_3$O$^-$, eventual traces of water might yield secondary by-products.

The degree of activation of most amino reactive payloads can be determined by spectroscopic assay by the so-called "Glycil-Glycine" test. The degree of PEG activation is determined by reacting an equimolar amount of glycine-glycine with the activated payload colorimetric assay of the unreacted dipeptide. This assay uses 2,4,6-trinitrobenzenesulfonic acid (TNBS) that reacts stoichiometrically with primary amino groups in alkaline medium to give a trinitrophenyl derivative absorbing at 420 nm. To determine the degree of activation of thiol reactive payload linker molecules (e.g., TOXIN-OPSS, TOXIN-Mal), the payload is mixed with an equimolar amount of a suitable molecule containing thiol (e.g., cysteine or glutathione). The unreacted thiol group is determined by Ellman assay based on the use of 5,5'-dithiobis (2-nitrobenzoic acid) (DTNB). The thiol group reacts with DTNB at neutral pH value to give 2-nitro-5-thiobenzoic acid (TNB), a derivative that absorbs at 412 nm. To measure the reactivity of NHS activated conjugation reagents, the reactivity of activated N-hydroxysuccinimidyl linker-payload can be easily evaluated by the following increase of UV absorbance at 260 nm due to the N-hydroxysuccinimide release performed at room temperature in borate buffer. Mass spectrometric molecular mass determination and NMR analyses of linker-payload agents can be conducted to assess identity of the conjugation reagent. For a control of low molecular weight reactive impurities in linker-payload batches MS analysis, RP-HPLC or NMR are useful to detect the presence of these substances.

Methods for the covalent conjugation of linker-payload with nucleases.

Endonuclease Protein covalent conjugation with linker payload molecules is achieved either by a direct chemical reaction between an amino acid residue and a suitable payload reagent or by an enzyme catalysed linkage. Several methods and strategies of conjugation are exemplified below.

Random amino payload molecule conjugation.

Protein primary amino groups are good nucleophiles and usually these groups are the most exploited for covalent conjugation of payload toxins in various drug conjugates. Coupling is conducted by reaction at mild basic media (pH 8-9.5). Lysins, commonly located on the protein surface, are relatively abundant amino acids, and easily accessible to activated linker-payload reagents (e.g., activated toxin-carboxylates and toxin-carbonates). Random payload conjugation at the level of this amino acid yields mixtures of different isomers and different degree of modification (Example 1 and Example Xenograft A549) . It must be noted that highly reactive conjugating linker-payload agents can target also, but at minor degree, other nucleophiles as the side chains of serine, threonine, tyrosine, and histidine. It is possible to cleave all these instable bonds by treating the conjugates mixture with hydroxylamine, thus improving the product homogeneity.

Protein N-terminal conjugation

For higher homogeneity of the drug conjugate, a selective payload coupling to the N-terminus of the protein can be archived, taking advantage of the different pKa values between the secondary-NH$_2$ of lysine and the tertiary-NH$_2$ of the N-terminus. By lowering the pH of the reaction mixture to about 5-6 all the secondaryamines will be protonated, while the tertiary-NH$_2$ group will still be available as free base for coupling. This method gives the best results using the low reactive aldehyde, propionaldehyde or butyraldehyde linkers. An unstable Schiff base is obtained in a first step, which in turn is reduced to a stable secondary amine.

Thiol payload molecule conjugation.

Cysteine residues are valuable targets for site-specific modification of proteins or peptides. They are present in the free form at a low natural abundance with respect the oxidized cystine species and these cysteines if present at all are frequently buried within globular protein structures with limited accessibility to chemical reagents. Taking this into consideration under appropriate conditions, free cysteins can be modified selectively, rapidly, quantitatively, in a reversible or irreversible fashion. By this promise of an easy coupling chemistry, By genetic engineering one or more cysteines were successfully introduced into the flexible single chain linker of the described CROMOC62 molecule position, which allowed for a highly efficient site-specific conjugation of the CROMOC62 molecule (Example 2 and Example Xenograft HCC). Payload-maleimide (Toxin-Mal), payload-vinylsulfone (Toxin-VS) or payload-iodo acetamide (Toxin-IA) derivatives are used to obtain irreversible and stable thioeter bonds between payload and nuclease. *Payload-orthopyridyl disulfide* (Toxin-OPSS) can be used which forms a disulfide-linkage with a cysteine on the protein. This linkage can be cleaved under reducing conditions, in contrast payload-Mal, which gives stable conjugates. PEG-IA and PEG-VS are less reactive, whereas PEG-Mal and PEG-OPSS yield quantitative protein modification. A strategy known in the art allows for direct conjugation to protein disulphide bridges, where the disulphide link is reduced, and the resulting free thiols are reacted with a special designed payload-monosulfone reagent to result in a stable three-carbon conjugated bridge.

Carboxylic group payload-conjugation.

Direct coupling of payload-$NH_2$ to activated protein carboxylic groups is complex and cannot be easily performed because it yields intra- or inter-molecular linkages within the protein amines. An original solution has been proposed by exploiting a payloadhydrazide, which is still reactive for carboxyl groups at low pH, whereas the protein amino groups are protonated.

Payload conjugation of proteins modified with aldehydic groups.

Carbonyl groups, absent in natural proteins, can be exploited for certain nucleophilic additions. They can be introduced by oxidation of a N-terminal threonine or serine using sodium periodate. The introduced aldehydic groups can react with an aminooxy-functionalized payload, to obtain a conjugate at the N-terminus of the protein. In some cases, when threonine or serine are not at the N-terminus, a comparably reactive group can be introduced by metal-catalysed oxidation. The later conditions, however, are potentially more harmful to the proteins.

Enzymatic approaches for nuclease-protein toxin conjugates (Grigoletto et al., Enzymatic Approaches to New Protein Conjugates. In Polymer-Protein Conjugates; Pasut, G., Zalipsky, S., Eds.; Elsevier, 2020; pp 271-295). Most of covalent conjugates between organic molecules and proteins approved for human use are based on chemical protocols. The demand for site-selective conjugates in which the small molecule is linked to a single, specific position on the protein sequence is strongly requested. Although some chemical methods meeting the site-selection criteria have been devised, their flexibility in terms of potential sites on the protein for the conjugation process and the number of techniques is limited. For this, enzymatic conjugation approaches can be employed. Enzymes can be used for endonuclease labelling given their advantageous unique properties of specificity and their catalytic function under physiological conditions.

Enzymatic methods for covalent conjugating small molecules to endonucleases can be divided at the high level into two groups, methods which can be used for non-tagged proteins, such as a transglutaminase or sialyltransferase enzymatic approach, and enzymatic coupling methods which require the presence of a specific amino acid substrate recognition site which must be added to the endonuclease as a tag. The former requires a specific site in a given endonuclease for a specific enzymatic conjugation method, the latter group mandatorily requires an additional step of a genetic engineering procedure to introduce an enzyme recognition tag. If the tag is functional, it will ideally allow for a precise site-specific conjugate. Naturally inserted amino acid tags into a protein can compromise its functionality. Tags are added after carefully studding nuclease structural information for designing a specific enzyme substrate recognition site, for the case of CROMOC these are engineered to the *C*- or *N*-terminus of the protein. Most of the endonuclease function as homo-dimers, such as for example restriction enzymes, and can be converted into functional single chain molecules by linking of the N-terminal head of the second nuclease unit to the C-terminal tail of the first unit of the dimer, employing a flexible single chain linker well known in the art (WO 2004/092194 A2). The single chain linker presents an ideal position to integrate a substrate recognition site. CROMOC62 and CROMOC63 insertions of maleimide coupling accessible cysteine into their single chain linkers, perfectly conserved the nuclease activity, arguing for single chain linkers besides N and C termini as a suitable position for substrate recognition site insertion into endonucleases. Other endonucleases represent natural occurring single chain molecules which show intramolecular dimerization, as is the case for CRISPR-Cas9 enzymes and intrinsic disordered structures which connect the two nuclease units might be approached for insertions of specific enzymatic tags. Frequently enzymatic approaches require specific functionalized small molecule payloads.

Transglutaminase for site specific conjugation of payload molecules.

The chemical reaction in the case of a small molecule payload catalyzed by TGases is the transfer reaction between the acyl moiety of the γ-carboxyamide group of a nuclease-bound glutamine residue ((Gln)acyl donor) and a linear primary amine ((Lys)acyl acceptors), which in nature is usually the ε-amino group of a toxin bound lysine residue. This reaction thus forms a crosslinking through an ε-(γ-glutamyl) lysine iso-peptide bond between the nuclease and a small molecule. Not all Gln residues in a protein may be recognized by a TGase. The recognition process determines the specificity of a TGase and generally only one or just a few glutamine residues among all those present in a globular protein are substrates of TGases, whereas the minimum requirement of the substrate is a structural flexibility of the sequence and the Gln residues that are substrate for the TGase need to be contained in a flexible or disordered portion of the nuclease. This class of enzymes is a versatile biochemical tool for the selective covalent conjugation of an endonuclease in the presence of a glutamine group for small molecules, that must contain a primary amino group. There is further a substantial advantage over random coupling because it is site directed, as under controlled conditions always the same, defined low number of Gln are conjugated in a protein. A fact that leads to defined, homogenous nuclease conjugate populations. This can be analyzed with methods well known in the art such as mass spectroscopy and protein sequencing methods. On purpose, specificity of mTGase can been enhanced by carrying out the catalysis in water/organic co-solvents reaction buffers, conditions that lead to an increase in the secondary structure elements of the protein substrates. When the enzymatic catalysis was conducted in buffers containing up to 50% (v/v) of ethanol, only a single conjugate isomer was obtained instead of a complex mixture of conjugates. This observation allows to tune the exact acceptor sites in a nuclease, by increasing the quantity of secondary structure elements, the organic solvent reduces the flexibility in some regions of the protein substrate thus abolishing the main prerequisite of backbone flexibility for an acyl donor which enables high site-selective specificity. Alternatively, the presence of flexible single chain linkers in many nucleases used for the invention allows to introduce one or more Gln residues, as an example but not for exclusion the cysteine residues in the single chain linker of CROMOC 62, and CROMOC63 are replaced by a glutamine acceptor site.

For the purification of the conjugated nuclease from the residual impurities a method as shown in Example 1 applying protein chromatography methods can be advantageously used. The highly basic DDM tag fused to the nuclease allows for stringent retention of the coupled proteins and separation of the mTGase and free toxins. Another method to form homogeneous monoconjugates with mTGase consists in the selective immobilization of the enzyme on the solid beads of an inert polysaccharide resin (Grigoletto A et al., J. Drug Target. 2017;25(9-10):856-64.). For this the immobilization was selectively directed to the *N*-terminal residue of mTGase in the attempt to avoid cross-linking and to minimize the loss of mTGase activity as the catalytic site is far from the N-terminus. mTGase immobilization made it possible to simply remove the enzyme from the reaction mixture at the end of the catalytic activity and to improve its stability.

Protein farnesyl transferase for site specific conjugation of payload molecules.

Protein farnesyl transferase (PFTase) is an enzyme that catalyzes the covalent attachment of a 15-carbon farnesyl isoprenoid from the farnesyl pyrophosphate (FPP) substrate to the cysteine of a conserved CaaX signal tetrapeptide through a thioether bond. The CaaX motif is found at the C-terminus of peptides and proteins. In the CaaX sequence, 'a' represents an aliphatic, non-polar and hydrophobic amino acid, X is usually serine, methionine, glutamine, alanine, or threonine. PFTase can tolerate a variety of FPP substrate analogues and different CaaX sequences without compromising its activity. Various isoprenoid surrogates containing azide and alkyne bio-orthogonal groups, aldehyde for aminoxy conjugation, biotin or aryl groups have been synthesized and proteins have been successfully tagged with these moieties, using PFTase. The PFTase-catalyzed transfer of synthetic alkyne or azide functionalized farnesyl analogues, followed by cycloaddition or ligation reactions have been used for the site-specific labeling of proteins with fluorophores and thus accordingly, toxin payloads are feasible for this method as well. Endonucleases can be genetically engineered at the C-termini with the CaaX motif. After prenylation, the modified protein can be treated with carboxypetidase to remove the aaX tripeptide of the CaaX sequence, in the end obtaining a protein with a single prenylcysteine modification and a single added cysteine at the C-terminus. This is another general method for C-terminal enzymatic site-specific modification of proteins with aldehyde groups, as most recombinant proteins are accessible for the fusion of a CaaX sequence. The DDM sequences can be flexible functionally fused to the N-terminus, into the single chain linker sequences, and to the C-terminus of the CROMOC nucleases. As such positioning CaaX might be or might not be fused onto the DDM C-terminal part.

Formyl-glycine-generating enzyme for site specific conjugation of payload molecules.

The natural role of formyl-glycine generating enzyme (FGE) is to convert a specific residue in the catalytic site of type I sulfatases to $C^\alpha$-formylglycine (FGly). The residue involved in the modification is usually a cysteine that undergoes oxidation, but it can also be a serine. The cysteine is part of the "sulfatase motif", a contiguous 13-amino acid sequence in which the highly conserved CXPXR (X is usually serine, threonine, alanine, or glycine) substrate-motif can be identified. FGE recognizes the substrate based entirely on a primary sequence and converts the first cysteine into the active FGly following a multistep redox process that requires calcium, molecular oxygen, and a reducing agent such as DTT.

The contained, so called "aldehyde tag modification" to a recombinant nuclease for a site-specific conjugation relies on the introduction of the CXPXR pentapeptide sequence, also referred to as the "aldehyde tag", into the protein backbone and in the subsequent oxidation of the cysteine in the tag by FGE. The newly installed aldehyde group can then be reacted with the nucleophile aminooxy attached small molecule payload, to create an oxime bond between the polymer and the protein. In this case, FGE does not directly mediate the conjugation of the payload with the protein, but it is prerequisite for introduction of a bio-orthogonal functionality at a specific site. Suggested variants of the aldehyde tag can be, the entire sulfatase motif composed of 13 residues (LCTPSRGSLFTGR) and a minimized 6-residue consensus sequence (LCTPSR) The target protein is co-expressed with a prokaryotic FGE from *Mycobacterium tuberculosis* to convert the cysteine residue in FGly during protein translation, with no need for otherwise additional ex *vivo* steps with recombinant enzymes.

Like other chemoenzymatic methods, aldehyde tag modification exploits the site-specific incorporation of a label into a protein mediated by an enzyme and the label's subsequent chemical modification. The specificity of FGE for such a short tag and the high homogeneity of the final product make the method an attractive option for nuclease-payload conjugation.

Lipoic acid ligase enzyme for site specific conjugation of payload molecules.

Lipoic acid ligase (LplA) is another enzyme that does not directly conjugate a payload to the endonuclease portion, but can introduce a new functional group, which allows for a selective, side reaction-free, conjugation step. In nature, LplA is an enzyme that catalyzes ATP-dependent covalent ligation of lipoic acid onto specific lysine side chains of three acceptor *E. coli* proteins involved in oxidative metabolism: pyruvate dehydrogenase (PDH), 2-oxoglutarate dehydrogenase, and the glycine cleavage system. The PDH complex, formed of multiple copies of three enzyme subunits, is responsible for the oxidative decarboxylation of the pyruvate to form acetyl-CoA and $CO_2$. The lipoic acid is attached to the protein through an amide bond between its carbonyl group and the ε-amino group of the protein lysine residue. LplA from *E. coli* catalyzes the reactions with the initial formation of the lipoyl-AMP intermediate from lipoic acid and ATP and then with the transfer of the intermediate to the apoprotein obtaining the lipoylated protein. Once the lipoylated protein is formed, lipoic acid retains an essential catalytic role in the metabolic pathways. LplA is a monomeric protein with a molecular weight of 38 kDa. The X-ray crystal structures of *E. coli* and LplA alone and in a complex with lipoic acid are available. It has also been demonstrated that LlpA and engineered variants are able to ligate unnatural moieties such as alkyl and aryl azide bearing a fluorophore or a photocrosslinker, replacing the lipoic acid with an engineered LplA acceptor peptide (LAP) tag, leading to covalently labeling any cell surface protein with any chemical probe thanks to a remarkable specificity. A 13-amino acid LAP-tag peptide (GFEIDKVWYDLDA), readily fuse-able to sites like C-N-termini or i.e., also to single chain regions or linkers by recombinant techniques, shows improved kinetic efficiency for LlpA's. The lysine residue in the LAP sequence is fundamental as it is the point of the lipoate attachment.

The site-specific conjugation of payloads to nucleases can be accomplished using LplA by a biorthogonal approach. LplA is used to attach an azide-containing moiety (10-azidodecanoic acid) to a LAP-tag substrate. LAP-tag containing endonuclease proteins are ligated using i.e, the enzyme mutant $^{w37v}$LplA and the ligated nuclease is then payload conjugated with a dibenzocyclooctyne functionalized payload-toxin (DBCO-toxin) via strain-promoted azide-alkyne cycloaddition (SPAAC) forming stable toxin-nuclease conjugates. Reactions can be as short as 30 min and conversion over 70% of the total protein.

Conjugate purification

A typical drug conjugate coupling reaction, especially random amino coupling, often yields in a mixture of heterogeneous compounds and therefore a purification step is of advantage. Likewise, also a selective conjugation reaction requires purification to eliminate unreacted amounts of protein, excesses of linker-payload, non-conjugated protein, and by-products. Depending on the biophysical properties of both, nuclease, and payload, various purification strategies are used well known in the art. Dialysis or ultrafiltration of the conjugation reaction mixture is used to remove low molecular weight components and for solvent exchange. Unreacted payload-compounds, unreacted nuclease proteins are achieved by chromatographic techniques with preferentially on-line detection systems. Proteins and most payloads and conjugates can be monitored by UV at 214 or 280 nm or by fluorescence ($\lambda_{ex}$ 295 nm, $\lambda_{em}$ 310 nm) .

Chromatographic purification For chromatographic purifications different principles are applied well described in (Mero, A.; Clementi, C.; Veronese, F. M.; Pasut, G. Covalent Conjugation of Poly(Ethylene Glycol) to Proteins and Peptides: Strategies and Methods. In Methods in Molecular Biology; 2011; Vol. 751, pp 95-129. https://doi. org/10.1007/978-1-61779-151-2_8), depending on the differences the conjugate brings about.

Ionic exchange chromatography (IEX)

When Toxins are coupled to endonuclease protein amino groups yields conjugates with an isoelectric point different from that of the starting native protein. Cation exchange chromatography (CEX) is the method of choice for separation of these proteins because it exploits differences in charges at the protein surface.

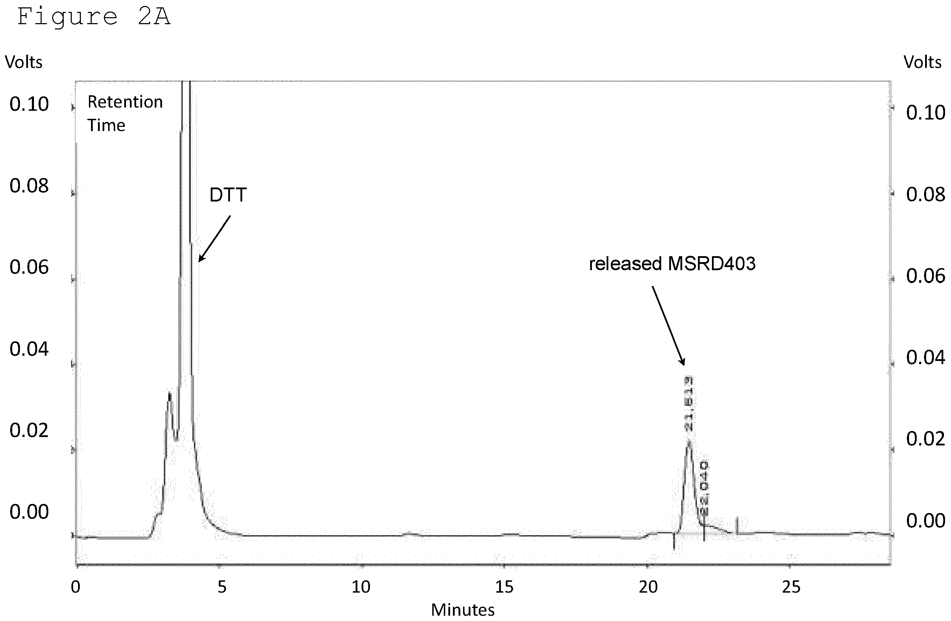

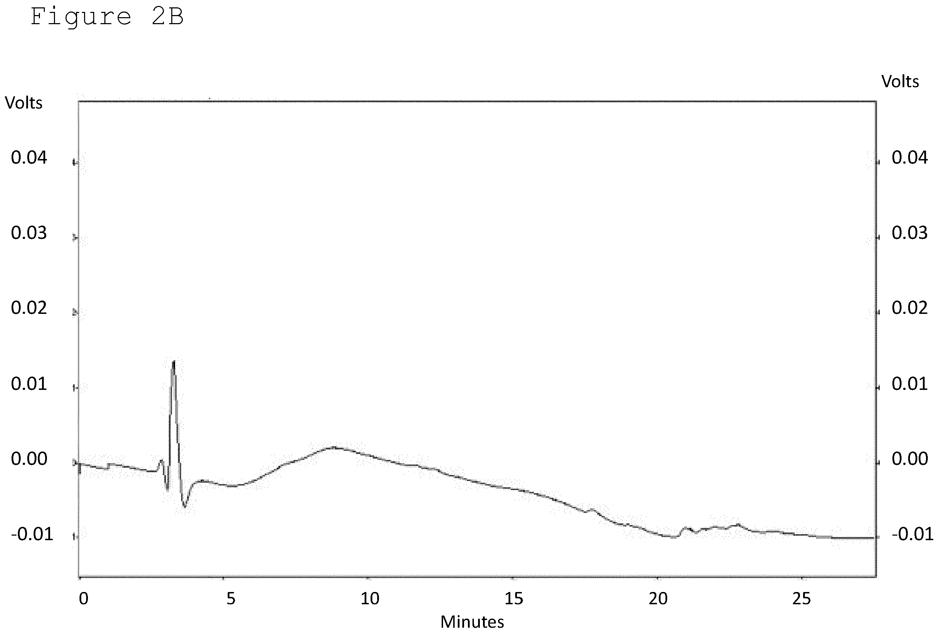

Anionic exchange chromatography (AEX). In this case the protein is eluted at higher pH value than its isoelectric point, this giving a negative net charge to the molecule. Reversed phase HPLC Reversed phase chromatography (RPC) is used for characterization of conjugated (i.e., Figure 2A and 2B) and purification of species due to its high resolution and to the possibility to use an on-line mass spectrometer detector. Sizeexclusion chromatography (SEC) allows separation of products based on differences in their hydrodynamic volumes. Unreacted Toxin can be separated by SEC. It is useful to remove low molecular weight impurities (by-products formed by hydrolysis of functionalized PEG, salts, solvents and other low molecular mass reagents).

Ultrafiltration/Diafiltration

A non-chromatographic step in the production of pharmaceutical proteins is the ultrafiltration/diafiltration operation. Diafiltration is a suitable process to exchange the buffer between chromatographic steps or to concentrate the conjugates at the desired final concentration. Usually, the membranes with the same cut-off used for native protein can be also employed to concentrate the conjugated sample. Regenerated cellulose and polyethersulfone membranes, with low protein binding and high protein retention can be employed.

Therefore, the present invention discloses the following embodiments:

1. Compound comprising or consisting of

(i) a polypeptide with the general formula (I):

$$X_0GX_1X_2GX_3X_4X_5GX_6X_7X_8GX_9X_{10}X_{11}X_{12}X_{13}X_{14},$$

wherein

G is glycine;
$X_0$ is present or not and, if present, is an amino acid linker;
$X_1$ is N or S, wherein N is asparagine and S is serine;
$X_2$ is S or T, wherein S is serine and T is threonine;
$X_3$ is present or not and, if present, is S, wherein S is serine;
$X_4$ is present or not and, if present, is G, wherein G is glycine;
$X_5$ is a basic polypeptide stretch consisting of 5 basic amino acid residues selected from R and K, wherein R is arginine and K is lysine, preferably wherein $X_5$ comprises at least 3 K amino acid residues, especially wherein $X_5$ is KKKKK or KRKKK;
$X_6$ is a basic amino acid residue selected from R and K, wherein R is arginine and K is lysine, preferably wherein $X_6$ is K;
$X_7$ is S or L, wherein S is serine and L is leucine;
$X_8$ is present or not and, if present, is a polypeptide stretch consisting of the amino acid sequence GLGS, wherein G is glycine, L is leucine and S is serine;
$X_9$ is a basic polypeptide stretch consisting of 3 basic amino acid residues selected from R and K, wherein R is arginine and K is lysine, preferably wherein $X_9$ comprises at least 2 K amino acid residues, especially wherein $X_9$ is KKK or KKR;
$X_{10}$ is present or not and, if present, is L or a polypeptide stretch consisting of the amino acid sequence DPL or DPC, wherein L is leucine, D is aspartic acid, P is proline, and C is cysteine;
$X_{11}$ is present or not and, if present, is a polypeptide stretch consisting of the amino acid sequence LR or GSGL, wherein L is leucine, R is arginine, G is glycine, and S is serine;
$X_{12}$ is present or not and, if present, is a basic polypeptide stretch with at least 20 % basic amino acid residues selected from K and R and at least a P residue, preferably wherein $X_{12}$ is selected from KYKPKL, KYKPKLGT, or $GX_3X_4X_5GX_6X_7X_8GX_9X_{10}$, wherein K, R, P, L, G, T, $X_3$, $X_4$, $X_5$, $GX_6$, $X_7$, $X_8$, $GX_9$, and $X_{10}$, are defined as above and wherein Y is tyrosine;
$X_{13}$ is present or not and, if present, is a polypeptide stretch consisting of the amino acid sequences GS, GST, GST, GSG, GSTG, or GSGL, wherein G, S, T and L are defined as above;
$X_{14}$ is present or not and, if present, is an amino acid linker; wherein the polypeptide has a length from 20 to 75 amino acid residues, preferably from 24 to 65 amino acid residues, especially from 25 to 60 amino acid residues; and

(ii) at least one payload molecule to be delivered into a biological cell,

wherein (i) and (ii) are covalently linked to each other, and

wherein (ii) is a single chain restriction enzyme and wherein the compound further comprises a toxin as second (ii) covalently coupled to (i), preferably in the order (ii) single chain restriction enzyme - (i) - (ii) toxin.

2. Compound according to embodiment 1, wherein $X_1$ is N, $X_2$ is S, $X_3$ is S, $X_4$ is G, $X_5$ is KKKKK or KRKKK, $X_6$ is K, $X_9$ is KKK or KKR, and $X_{10}$ is L or a polypeptide stretch consisting of the amino acid sequence DPL or DPC, wherein N, S, G, R, L, D, P and C are defined as above.

3. Compound according to embodiment 1 or 2, wherein (ii) is selected from the group

GNSGSGKRKKKGKGLGKKRDPCLRKYKPKLGTGSTG(HHHHHH) (DDM11),
GNSGKRKKKGKGSGLGSGKKRDPCLRKYKPKLGSTG(HHHHHH) (DDM18),
GNSGSGKRKKKGKGLGKKRDPCLRKYKPKLGTGSTG(HHHHHH) (DDM21),
GNSGSGKRKKKGKGSGLGSGKKRDPLGSTG(HHHHHH) (DDM26),
GNSG(HHHHHH)GSTGGKRKKKGKGSGLGSGKKRDPL (DDM27),
GNSGSGKRKKKGKGSGLGSGKKKDPLGSTG(HHHHHH) (DDM28),
GNSG(HHHHHH)GSTGGKRKKKGKGSGLGSGKKKDPL (DDM29),
GNSGSGKKKKKGKGSGLGSGKKRDPLGSTG(HHHHHH) (DDM30),
GNSG(HHHHHH)GSTGGKKKKKGKGSGLGSGKKRDPL (DDM31),
GNSGSGKKKKKGKGSGLGSGKKKDPLGSTG(HHHHHH) (DDM32),
GNSG(HHHHHH)GSTGGKKKKKGKGSGLGSGKKKDPL (DDM33),
GNSGSGKKKKKGKGSGLGSGKKKDPL (DDM34),
GNSGSGKKKKKGKGSGLGSGKKKLGSTG(HHHHHH) (DDM35), GNSGSGKKKKKGKGSGLGSGKKKDPL
GSGLGSGKKKKKGKGSGLGSGKKKDPLGST (DDM36), and

```
GNSGSGKKKKKGKGSGLGSGKKKDPLGSGLGSGKKKKKGKGSGLGSGKKKDPLGSTG(HHHHHH)
GST (DDM44),
```

wherein (HHHHHH; a histidine tag) may be present or not; preferably
GNSGSGKRKKKGKGLGKKRDPCLRKYKPKLGTGSTG (DDM11),
GNSGKRKKKGKGSGLGSGKKRDPCLRKYKPKLGSTG (DDM18),
GNSGSGKRKKKGKGLGKKRDPCLRKYKPKLGTGSTG (DDM21),
GNSGSGKRKKKGKGSGLGSGKKRDPLGSTG (DDM26),
GNSGGSTGGKRKKKGKGSGLGSGKKRDPL (DDM27),
GNSGSGKRKKKGKGSGLGSGKKKDPLGSTG (DDM28),
GNSGGSTGGKRKKKGKGSGLGSGKKKDPL (DDM29),
GNSGSGKKKKKGKGSGLGSGKKRDPLGSTG (DDM30),
GNSGGSTGGKRKKKGKGSGLGSGKKRDPL (DDM31),
GNSGSGKKKKKGKGSGLGSGKKKDPLGSTG (DDM32),
GNSGSGKKKKKGKGSGLGSGKKKDPL (DDM34),
GNSGSGKKKKKGKGSGLGSGKKKDPLGSGLGSGKKKKKGKGSGLGSGKKKDPLGST (DDM36),
and GNSGSGKKKKKGKGSGLGSGKKKDPLGSGLGSGKKKKKGKGSGLGSGKKKDPLGSTGGST (DDM44),
especially
GNSGSGKKKKKGKGSGLGSGKKKDPLGSGLGSGKKKKKGKGSGLGSGKKKDPLGST (DDM36), and GNS
GSGKKKKKGKGSGLGSGKKKDPLGSGLGSGKKKKKGKGSGLGSGKKKDPLGSTGGST (DDM44).

4. Compound according to any one of embodiments 1 to 3, wherein (ii) is a chemotherapeutic molecule, a cytotoxic molecule, a DNA damaging molecule, an anti-metabolite molecule, a therapeutic molecule, a small molecule with therapeutic effect inside a biological cell, a DNA molecule, an RNA molecule, an antibody molecule or an antibody derivative having an antibody-like function, a restriction endonuclease, a nicking enzyme, or DNA- or RNA-dependent endonucleases which show double strand breaking nuclease double strand/single strand breaking activity or no nuclease activity, more preferred wherein the payload molecule is a class II restriction endonuclease, such as PvuII, EcoRV, PvuII, HinfI, or a sc homodimer, a subunit or a functional fragment thereof; especially wherein the payload molecule is selected from the group

microtubule inhibitors, especially auristatin, maytansinoids, tubulysins; cytotoxic small molecules with a molecular mass of 300 to 1300 Daltons, Dimethyl-SGD-1882,CAS1222490-34-7, SN-38, 7-Ethyl-10-hydroxycamptothecin, CAS86639-52-3, Duocarmycin, CAS118292-34-5, Tubulysin-beta, Cas 1943604-24-7, Tubulysin A(TubA) CAS 205304-86-5, Tubulysin, Tubulysin B, M, IM1, IM2, IM3, CAS205304-86-5, Tubulysin C, Tubulysin D, Tubulysin E, Tubulysin F, Tubulysin G, Tubulysin H, Tubulysin I, Maytansinoid AP-3 CAS 66547-09-Auristatin E CAS 160800-57-7, Auristatin F CAS163768-50-1, Dolastatin 10 CAS10417-88-4, Maytansinol CAS57103-68-1, Monomethyl auristatin D (MMAD) CAS203849-91-6, Monomethylauristatin F(MMAF) CAS745017-94-1, Monomethylauristatin E (MMAE) CAS474645-27-7, DM1 Maytansinoid CAS139504-50-0, DM4 Maytansinoid CAS799840-96-3, Maytansine DM3 CAS 796073-54-6, Pyrrolobenzodiazepine (PBDs), Rebeccamycin Sandramycin CAS100940-65-6,10-Deacetyl-7-xylosyl paclitaxel CAS90332-63-1, alpha-Amanitin CAS23109-05-9, gamma-Amanitin, Ansamitocin P-3 CAS66584-72-3, Calicheamicin CAS108212-75-5,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIY,

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIY,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGGSGGSSHPDLNKLLELWPHIQEY
QDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIY,

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGGSGGSSHPDLNKLLELWPHIQEY
QDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIY,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCSGGSSHPDLNKLLELWPHIQEY
QDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIY,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCSGGSSHPDLNKLLELWPHIQE
YQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIY,

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCSGGSSHPDLNKLLELWPHIQEY
QDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIY,

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCSGGSSHPDLNKLLELWPHIQE
YQDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIY,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCCSGGSSHPDLNKLLELWPHIQ
EYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIY,

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCCSGGSSHPDLNKLLELWPHIQ
EYQDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIY,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIY**GSGGCAACSGGS**SHPDLNKLLELWPHI
QEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

and

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIY**GSGCAACAACSGS**SHPDLNKLLELWPH
IQEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST;

wherein in a preferred
compound the (single chain) nuclease moiety (as a first payload (ii)) is connected to the N-terminus of the polypeptide (i) (which is preferably a CROMOC molecule as explicitly disclosed and referred to herein), wherein a toxin (as a second payload molecule (ii) is connected to an amine group (or to a cysteine residue) of the polypeptide (i); wherein the moieties are preferably connected by linker molecules (especially cleavable linkers), said preferred compound being preferably PEGylated, especially at the N-terminus of the polypeptide (i) or at a cysteine residue.

5. Compound according to any one of embodiments 1 to 4, wherein the compound additionally comprises a homopolymer moiety covalently attached to the polypeptide with the general formula (I) or with the payload molecule (ii), preferably wherein the homopolymer is selected from the group of polyethylene glycol (PEG), especially a PEG with a MW of 5 to 30 kDa; dextran, polysialic acids, hyaluronic acid, dextrin, hydroxyethyl starch, or poly(2-ethyl 2-oxazoline.

6. Compound according to any one of embodiments 1 to 5, wherein the compound additionally comprises a restriction endonuclease as a payload molecule to be delivered into a biological cell covalently attached to the polypeptide with the general formula (I), preferably a class II restriction endonuclease, especially a PvuII restriction endonuclease or a derivative thereof wherein the derivative is a single chain PvuII restriction endonuclease, such as the derivative comprising the amino acid sequence

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIY,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGG,

SGGSSHPDLNKLLELWPHIQEYQDLALKHGINDIFQDNGGKLLQVLLITGLTVLPGREGNDAVDN
AGQEYELKSINIDLTKGFSTHHHMNPVIIAKYRQVPWIFAIYRGIAIEAIYRLEPKD-
LEFYYDKWERKWYSDGHKDINNPKIPVKYVMEHGTKIY,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGNSG,

MSHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIY,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGC,

CSGGSSHPDLNKLLELWPHIQEYQDLALKHGINDIFQDNGGKLLQVLLITGLTVLPGREGNDAVD
NAGQEYELKSINIDLTKGFSTHHHMNPVIIAKYRQVPWIFAIYRGIAIEAIYRLEPKD-
LEFYYDKWERKWYSDGHKDINNPKIPVKYVMEHGTKIY,

MSHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCSGGSSHPDLNKLLELWPHIQEY
QDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIY,

MSHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGSGGSSHPDLNKLLELWPHIQEYQ
DLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIY,

MSHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGSGGS,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGSGGS,

GSGGSGGSSHPDLNKLLELWPHIQEYQDLALKHGINDIFQDNGGKLLQVLLITGLTVLPGREGND
AVDNAGQEYELKSINIDLTKGFSTHHHMNPVIIA-
KYRQVPWIFAIYRGIAIEAIYRLEPKDLEFYYDKWERKWYSDGHKDINNPKIPVKYVMEHGTKIY
,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIY**GSGGCAACSGGS**SHPDLNKLLELWPHI
QEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW

```
YSDGHKDINNPKIPVKYVMEHGTKIYGNSGSGKKKKKGKGSGLG-
SGKKKDPLGSGLGSGKKKKKGKGSGLGSGKKKDPLGST,
```

or

```
SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGCAACAACSGSSHPDLNKLLELWPH
IQEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST.
```

7. Compound according to any one of embodiments 1 to 6, wherein the polypeptide with the general formula (I) is covalently linked to another moiety by a linker, which is a cleavable or a non-cleavable linker, wherein the another moiety is preferably a payload molecule to be delivered into a biological cell, a labelling group, and/or a homopolymer.

8. Compound according to any one of embodiments 1 to 7, wherein the compound further comprises a capping group, preferably an alkoxy, especially a methoxy, ethoxy, propoxy, or butoxy group; a halogen atom; or a tosylate; isocyanate, hydrazine hydrate, maleimide, orthopyridyl disulfide, N-succinimidyloxy, sulfo-N-succinimidyloxy, 1-benzotriazol, 1-imidazolyloxy, p-nitrophenyloxy, or aldehyde.

9. Compound according to any one of embodiments 1 to 8, wherein the compound further comprises a linker, wherein the linker is an amino acid linker of 1 to 20 amino acid residues in length, preferably from 1 to 15 amino acid residues, more preferred from 7 to 13 amino acid residues, especially of 8 to 12 amino acid residues.

10. Compound according to any one of embodiments 1 to 9, wherein the compound further comprises a linker, wherein the linker is an amino acid linker, selected from a linker comprising or consisting of cysteine, serine or glycine residues, preferably a single or two adjacent cysteine residue (s); an amino acid sequence comprising glycine and serine residues or comprising or consisting of the amino acid sequences GSG, SGG, GGC, GCS, CSG, GGS, GSGG, SGGS, GSGGC, CSGGS, GSGGSGGS, GSGGCCSGGS, GSGGCCCSGGS, GSGGCSGGS, GSGGCAACSGGS or GSGCAACAACSGS.

11. Compound according to any one of embodiments 1 to 10, wherein the compound further comprises as payload two monomeric subunits or parts thereof of a type II restriction endonuclease, preferably, two monomeric alpha-helical subunits of a type II restriction endonuclease covalently connected by a linker to the polypeptide with the general formula (I).

12. Compound according to any one of embodiments 1 to 11, wherein the compound further comprises as payload two monomeric alpha-helical subunits of PvuII, preferably wherein the two monomeric subunits or parts thereof of the type II restriction endonuclease PvuII are covalently connected by a linker to the polypeptide with the general formula (I).

13. Compound according to any one of embodiments 1 to 12, wherein the general formula (I) contains a single arginine and/or a single cysteine or not more than two arginine residues and/or not more than two cysteine residues

14. Compound according to any one of embodiments 1 to 13, wherein the general formula (I) lacks arginine and/or cysteine, preferably wherein the general formula (I) lacks arginine and cysteine.

15. Compound according to any one of embodiments 1 to 14, comprising at least two polypeptides with the general formula (I).

16. Compound according to any one of embodiments 1 to 15, wherein the compound is selected from the group GNSGSGKKKKKGKGSGLGSGKKKDPL (DDM34) and GNSGSGKKKKKGKGSGLGSGKKKDPLGSGLGSGKKKKKGKGSGLGSGKKKDPLGST (DDM36).

17. Compound according to any one of embodiments 1 to 16, wherein the polypeptide with the general formula (I) is covalently coupled to the C-terminus of a payload molecule to be delivered into a biological cell, preferably a compound selected from the group

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGNSGSGKKKKKGKGSGLGSGKKKDPLG
SGLGSGKKKKKGKGSGLGSGKKKDPLGST,

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGNSGSGKKKKKGKGSGLGSGKKKDPLG
SGLGSGKKKKKGKGSGLGSGKKKDPLGST,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGGSGGSSHPDLNKLLELWPHIQEY
QDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGGSGGSSHPDLNKLLELWPHIQEY
QDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCSGGSSHPDLNKLLELWPHIQEY
QDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-

LEFYYDKWERKWYSDGHKDINNPKIPVKYVMEHGTKIYGSGGCCSGGSSHPDLNKLLELWPHIQE
YQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCSGGSSHPDLNKLLELWPHIQEY
QDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCSGGSSHPDLNKLLELWPHIQE
YQDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCCSGGSSHPDLNKLLELWPHIQ
EYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

SHPDLNKLLELWPHIQEYQDLALKHGINDIFQDNGGKLLQVLLITGLTVLPGREGNDAVDNAGQE
YELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCCSGGSSHPDLNKLLELWPHIQ
EYQDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIY**GSGGCAACSGGS**SHPDLNKLLELWPHI

QEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST,

and

SHPDLNKLLELWPHIQEYQDLALKHGINDIFQDNGGKLLQVLLITGLTVLPGREGNDAVDNAGQE
YELKSINIDLTKGFSTHHHMNPVIIAKYRQVPWIFAIYRGIAIEAIYRLEPKD-
LEFYYDKWERKWYSDGHKDINNPKIPVKYVMEHGTKIY**GSGCAACAACSGS**SHPDLNKLLELWPH
IQEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERKW
YSDGHKDINNPKIPVKYVMEHGTKIYGNSGSGKKKKKGKGSGLG-
SGKKKDPLGSGLGSGKKKKKGKGSGLGSGKKKDPLGST.

18. Compound according to any one of embodiments 1 to 17, wherein the polypeptide with the general formula (I) is covalently coupled to the C-terminus of a payload molecule to be delivered into a biological cell which is a class II restriction endonuclease, preferably PvuII or a subunit thereof, wherein the payload molecule is optionally attached at its C-terminus to a linker molecule comprising 8 to 12 amino acids comprising or consisting of glycine, serine and/or cysteine residues, and wherein the polypeptide with the general formula (I) is covalently coupled to the C-terminus of the payload molecule or the linker molecule, wherein the compound comprises one or two payload molecule(s), optionally separated by the linker molecule comprising 8 to 12 amino acids comprising or consisting of glycine, serine and/or cysteine residues.

19. Compound according to any one of embodiments 1 to 18, wherein the polypeptide with the general formula (I) is covalently coupled to the C-terminus of a payload molecule to be delivered into a biological cell, wherein the compound comprises a first payload molecule which is the first subunit of PvuII linked at its C-terminus to a linker molecule comprising 8 to 12 amino acids comprising or consisting of glycine, serine and/or cysteine residues, wherein the linker molecule is attached at its C-terminus to the second subunit of PvuII and wherein the polypeptide with the general formula (I) is covalently coupled to the C-terminus of the second subunit of PvuII.

20. Compound according to any one of embodiments 1 to 19, for use in the treatment of a tumour patient, preferably wherein the tumour patient is suffering from neuroblastoma, colon carcinoma, hepatocellular carcinoma, Small Cell Lung carcinoma, ovarian carcinoma, lung carcinoma, bladder carcinoma, prostate carcinoma, uterus carcinoma, cervix carcinoma, placental carcinoma, breast carcinoma, kidney carcinoma, liver carcinoma, pancreas carcinoma, muscle carcinoma, skin carcinoma, connective tissue carcinoma, bone carcinoma, and any of these carcinomas wherein the patient has already developed metastases, especially for the treatment of a patient having neuroblastoma, colon carcinoma, hepatocellular (liver) carcinoma, Small Cell Lung carcinoma, lung carcinoma, breast carcinoma, pancreas carcinoma, and any of these carcinomas wherein the patient has already developed metastases.

21. Method of treatment of a tumour patient wherein an effective amount of the compound according to any one of embodiments 1 to 19 is administered to a patient in need thereof, preferably wherein the tumour patient is suffering from neuroblastoma, colon carcinoma, hepatocellular carcinoma, Small Cell Lung carcinoma, ovarian carcinoma, lung carcinoma, bladder carcinoma, prostate carcinoma, uterus carcinoma, cervix carcinoma, placental carcinoma, breast carcinoma, kidney carcinoma, liver carcinoma, pancreas carcinoma, muscle carcinoma, skin carcinoma, connective tissue carcinoma, bone carcinoma, and any of these carcinomas wherein the patient has already developed metastases, especially for the treatment of a patient having neuroblastoma, colon carcinoma, hepatocellular (liver) carcinoma, Small Cell Lung carcinoma, lung carcinoma, breast carcinoma, pancreas carcinoma, and any of these carcinomas wherein the patient has already developed metastases.

22. Use of a compound according to any one of embodiments 1 to 19 for the manufacture of a medicament, preferably for the treatment of a tumour patient, more preferred for the treatment of a patient having neuroblastoma, colon carcinoma, hepatocellular carcinoma, Small Cell Lung carcinoma, ovarian carcinoma, lung carcinoma, bladder carcinoma, prostate carcinoma, uterus carcinoma, cervix carcinoma, placental carcinoma, breast carcinoma, kidney carcinoma, liver carcinoma, pancreas carcinoma, muscle carcinoma, skin carcinoma, connective tissue carcinoma, bone carcinoma, and any of these carcinomas wherein the patient has already developed metastases, especially for the treatment of a patient having neuroblastoma, colon carcinoma, hepatocellular (liver) carcinoma, Small Cell Lung carcinoma, lung carcinoma, breast carcinoma, pancreas carcinoma, and any of these carcinomas wherein the patient has already developed metastases.

23. Pharmaceutical preparation comprising a compound according to any one of embodiments 1 to 19 and a protein kinase inhibitor (PKI), preferably a DNA-dependent PKI, more preferred a Non Homologous End Joining (NHEJ) and V(D)J repair factor DNA-dependent PKI, especially 7-Methyl-2-[(7-methyl[1,2,4]triazolo[1,5-a]pyridin-6-yl)amino]-9-(tetrahydro-2H-pyran-4-yl)-7,9-dihydro-8H-purin-8-one (AZD7648).

The present invention is further disclosed by the following examples and the figures, yet without being limited thereto.

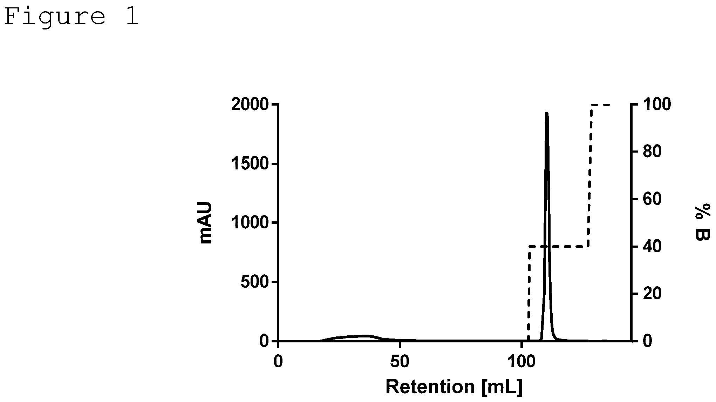

Figure 1 shows the chromatogram of the purification profile of the CROMOC62-SMCC-DM1 sample. Absorbance at 280 nm (solid line), conductivity profile (dotted line,) the percentage of elution-buffer (striped line).

## Examples

Abbreviations

| | |
|---|---|
| CV | Column Volume |
| DPR | Drug to Protein Ratio |
| ε | Molar Extinction Coefficient |
| EC50 | Half Maximal Effective Concentration |
| FPLC | Fast Protein Liquid Chromatography |
| mAU | Milli-Absorbance Units |
| MWCO | Molecular Weight Cut Off |
| OD | Optical Density |
| PBS | Phosphate Buffered Saline |
| PEG | Polyethylene glycol |
| RT | Room Temperature |
| VS-BU | Valdospan Buffer |
| | |

Example 1

Preparation of CROMOC62 DM1 nuclease drug conjugates CROMOC62-SMCC-DM1conjugation

This Example demonstrates, how a CROMOC nuclease can be highly efficiently conjugated using the high-toxic DM1 Maytansinoid small molecule employing a stable succinimidyl 4-(N-maleimidomethyl) cyclohexane-1-1carboxylate (SMCC) linker. The procedure is described for CROMOC62 and applies to other CROMOC molecules as well where the same procedure can be used. In using this coupling procedure, a random coupling of surface exposed amines can be covalently linked with toxins. As well known in the art this procedure applies also for cleavable linkers as well, whenever a secondary nitrogen of the carrier molecule is conjugated. This example also demonstrates that the used coupling reaction is efficient for long chain polyethylene-glycol modified (PEGylated)nucleases. The later advantageously facilitates a coupling process with high toxic substances, reducing toxin exposure to a minimum as compared to a PEGylation process that needs to be conducted after the toxin is linked to a protein.

Method for coupling of SMCC-DM-1 to CROMOC62

PEGylated CROMOC62 endonuclease prepared as described in PCT/EP2022/086215 stored in PBS pH= 7.4 at minus 80°C was thawed at 25°C and adjusted with PBS to pH= 8 for the SMCC-DM-1 conjugation reaction. For the conjugation reaction an excess of four molecular equivalents of SMCC-DM1 (**Table 1**) to CROMOC62 is used. CROMOC62 exhibits a molecular mass of 4,2400 g/mol and SMCC-DM1 exhibits a molecular mass of 1072,6 g/mol. The CROMOC62 nuclease used was modified with a 30kDa long single chain poly-ethylene-glycol covalently coupled to a unique cysteine residue in the CROMOC62 molecule. For the conjugation reaction the following reagents were added using a sequential order as follows. To ten milligrams of CROMOC62 in 1730 $\mu$l of PBS pH7.4 was diluted with 1267,7 $\mu$l of PBS pH 8.0. Following dilution 232.1 $\mu$l of DMSO was added and 1.01 mg of SMCC-DM1 in 101.2 PBS pH8.0 was added to start the reaction. After gently mixing, the reaction was allowed to proceed for exact one hour at 25°C. After this, for a purification of the conjugate from the residual free SMCC-DM1 toxin, the reaction mixture was diluted 1:4 with VS-BU-004 **(Table 1)** to lower the salt concentration to 0.2 M. This diluted reaction with a final protein concentration of 3.0 mg/ml was purified by cation exchange chromatography on a CIEX Poros XS **(Table 2)** column.

**Table 1** Reagents, Downstream processing buffers

| Reagents - DOWNSTREAM PROCESSING - Buffers | |
|---|---|
| Buffer Name | Provider respectively - Purpose - Compounds |
| VS-BU-004 | CATION CHROMATOGRAPHY<br>20 mM NaH2PO4/Na2HPO4 pH 7.0, 1 mM EDTA to volume with Type 1 H20, filtered 0.2 $\mu$m |
| VS-BU-005 | CATION CHROMATOGRAPHY<br>20 mM NaH2PO4/Na2HPO4 pH 7.0, 1 mM EDTA, 2 M NaCl to volume with Type 1 H20, filtered 0.2 $\mu$m |
| REAGENTS | |
| Name | Provider, Cat. No., Lot No. |
| SMCC-DM1 | MedChemExpress, cat.# HY-101070/CS-6309, lot#: 190165, |
| NaCl | Carl Roth, cat.# 3957.4, lot# 052318750 |
| NaOH | Carl Roth, cat.# 8655.3, lot# 301311601 |
| Na$_2$HPO$_4$ | Sigma-Aldrich, cat.#: 30412-1KG, lot# SZBA2860V |
| NaH$_2$PO$_4$ | Fluka, cat.# 71504, lot# 1388449 |
| EDTA | Merck, cat.# 324503-1KG, lot# 3468653 |
| PBS | Biowest, cat.# L0615-500, lot# MS01FA |

**Table 2** Material columns and devices for CROMOC62-SMCC-DM1 drug conjugate purification

| MATERIAL | |
|---|---|
| Name | Provider, Cat.#, Lot# |
| AKTA™ pure 150L | Cytiva, cat.# 29046665, Serial# 2845794 |
| POROS XS prepacked column (5ml) | Thermo-Fisher Scientific, cat.# A36638, lot# WG330617, CV is 5 ml |
| Centrifuge 6K15 | Sigma-Aldrich |
| Rotor 11650 404/G | Sigma-Aldrich |
| Buckets 13650 137/H | Sigma-Aldrich |
| Amicon® Ultra 15 mL Centrifugal Filters, Ultracel-30K MWCO | Merck cat.#: UFC903024, lot#: 0000177030 |
| Syringe filter Minisart Filter unit | Sartorius Stedim cat.#: 16534-K, lot#: 211749103 |
| | |

(continued)

| COLUMNS | | | |
|---|---|---|---|
| Product Name | Manufacturer Lot | Column Data | Flow Data |
| CIEX Poros XS | cat.# A36638, lot# WG330617 Thermo-Fisher Scientific, 3747 N. Meridian Rd., Rockford IL 61101 U.S.A | CV = 5 ml<br><br>Bed Height = 10 cm<br>Column Diameter = 8 mm | 215 cm/hr<br>2.8 min<br><br>1.8 ml/min |

Method of purification of CROMOC62-SMCC-DM-1 by cation exchange chromatography

Prior to by cation exchange chromatography FPLC purification, the Äkta System pumps and inlets were equilibrated as follows: pump A, inlet 1 = VS-BU-004, pump A, inlet 2 = VS-BU-004, pump B, inlet 1 = VS-BU-005, pump B, inlet 2 = VS-BU-005.

The sample was loaded onto a pre-equilibrated (VS-BU-004) POROS XS 5 ml column (Thermo Fisher Scientific) using pump A, inlet 2. The tube was flushed with 30 ml buffer VS-BU-004. After a wash with 4 CV (20 ml) VS-BU-004, the protein was eluted using a 2-step gradient: 1.)40 % buffer VS-BU-005 using pump B, inlet 20.8 M NaCl, elution volume: 5 CV (25 ml) 2.)100 % buffer VS-BU-005 using pump B, inlet 2.2 M NaCl, elutes non-native isomeric forms, elution volume: 2 CV (10 ml)

Run parameters were: Constant flow rate: 215 cm/hr (1.8 ml/min). All reagents and buffers **Table 1 and Table 2.** Following product turnover, the System and instrument cleaning steps were as follows. After removing the column from the system, to avoid endotoxin contamination of the sample in the next chromatographic step, all pump inlets were brought to 0.5 M NaOH and incubated for 30 minutes before being rinsed extensively with type 1 $H_2O$.

Buffer exchange and concentration adjustment of the conjugated cation exchange purified CROMOC62-SMCC-DM1 drug conjugate

The buffer of the CROMOC62-SMCC-DM1 conjugate (the sample) was changed by repeated cycles of concentration and dilution using one AMICON spin filter (15ml) at 4000g, 4°C **(Table 2).** Hereby the process was stopped every 10 minutes and the samples were inverted three times to avoid a concentration gradient within the filter. A protein concentration >3 mg/ml was avoided to exceeded during the process as follows. The sample was diluted in PBS 1:5 and concentrated again three times (total dilution factor 1:125) to obtain a residual VS-BU-004/005 concentration of <1 %. The sample was sterile filtered using a 0.22 $\mu$m syringe filter. The $OD_{280}$ of the concentrated sample was measured using PBS as a blank on a DENOVIX DS II optical photometer. Protein concentration was calculated from the theoretical molar extinction coefficient, neglecting the minimal absorption of PEG at 280 nm.

Protein long term storage

The sample was aliquoted into pyrogen-free 1.5 ml Eppendorf tubes (LPS and RNase free) and frozen at -80°C.

Results

SMCC-DM-1 CROMOC62 Cation Exchange Chromatography

**Figure 1** shows the chromatogram of the purification profile of the CROMOC62-SMCC-DM1 sample. Absorbance at 280 nm (solid line), conductivity profile (dotted line,) the percentage of elution-buffer (striped line). During a two-step elution with 40% respectively 100% of high salt elution-buffer VS-BU-005 **(Table 1),** CROMOC62-SMCC-DM1 elutes with the main peak fraction at 40% corresponding to 0.8 M NaCl. Residual, free SMCC-DM1 separates in the flow trough.

Determining of the CROMOC62 SMCC-DM1 protein concentration and drug to protein ratio (DPR)

Spectrophotometric Analysis

Absorption of the final protein conjugate was determined by measuring the optical density (OD) of the sample at 252nm and 280nm using a nano spectrophotometer. As both, CROMOC62 and SMCC-DM1 show overlapping absorption at the absorption maximum of each other, concentrations of CROMOC62 protein and conjugated SMCC-DM1 toxin were calculated using the equation system for the Extinction coefficients and measured Absorption values as follows:

$$OD(252) = c(CROMOC) * \varepsilon(CROMOC, 252) + c(DM1) * \varepsilon(DM1, 252)$$

$$OD(280) = c(CROMOC) * \varepsilon(CROMOC, 280) + c(DM1) * \varepsilon(DM1, 280)$$

| Extinction coefficients ($\varepsilon$) | | | Absorption | |
|---|---|---|---|---|
| Name | 252 nm | 280 nm | 252 nm | 280 nm |
| CROMOC62 | 26372 | 73800 | 3.719 | 4.422 |
| SSC-DM1 | 24201 | 5065.2 | | |

The measured concentrations were used to determine the drug to protein ratio (DPR) using the formula:

| Concentration and DPR | | | | | | |
|---|---|---|---|---|---|---|
| Name | CROMOC [μM] | DM1 [μM] | DPR | Concentration [mg/ml] | Volume [ml] | Amount [mg] |
| CROMOC62 | 53.4 | 95.5 | 1.79 | 2.24 | 3.6 | 8.06 |

$$DPR = c(DM1)/c(CROMOC)$$

Pierce 660nm Protein Assay Analysis

Sample concentrations were confirmed independently by using the Pierce660nm Protein Assay (Thermo Fisher Scientific: cat#: 1861426, lot#: WJ335666) in the microtiter plate application according to the corresponding protocol. Pierce 660nm Protein Assay revealed a protein concentration of 2.28 mg/ml.

Example 2

Preparation of CROMOC62 and CROMOC70 nuclease Tubulysin MSRD403-SS-Py drug conjugates

An activated Tubulysin A derivative MSRD403-SS-Py (MW941Da) WO2004/005327, WO 2008/138561A1 was coupled to the single cysteine in CROMOC62 using a molar ratio of 1:1 between the toxin and the protein. The amount of coupled MSRD403 in the dialysis purified conjugate was determined, based on a calibration curve, by RP-HPLC after release of MSRD403 by DTT treatment **Figure 2A and Figure 2B.**
Figure 2A shows the RP-HPLC profile of 8.10 mg/ml of CROMOC62-SS-MSRD403 under reducing conditions, the sample was reduced prior to loading with 10mM DTT. Reversed phase (RP) Phenomenex C18 (4.6x250 mm) 00G-4053-E0. Buffers and elution gradient was Buffer A: 10mM (NH4)2CO3, Buffer B: MeCN, 40% B in 10', 52%B 5' 70%B 3' 90%B 5' 90%B at a flow rate of 1 ml/min. and 254nm UV detection using a 20ul sample loop. Figure 2B: The CROMOC62-SS-MSRD403 was analyzed by RP-HPLC under non-reducing conditions. RP-HPLC without DTT treatment to verify the absence of free toxin. Conditions as in Figure 2A. Final CROMOC62-SS-MSRD403 was 1.0mg/ml and had a MSDR403 loading of 0.00622 mg/ml. This preparation was used for Example 8.
+Protein concentrations were determined using the average between the values obtained from the absorbance measurement at 280 nm and from a BCA protein determination assay.

Example 3

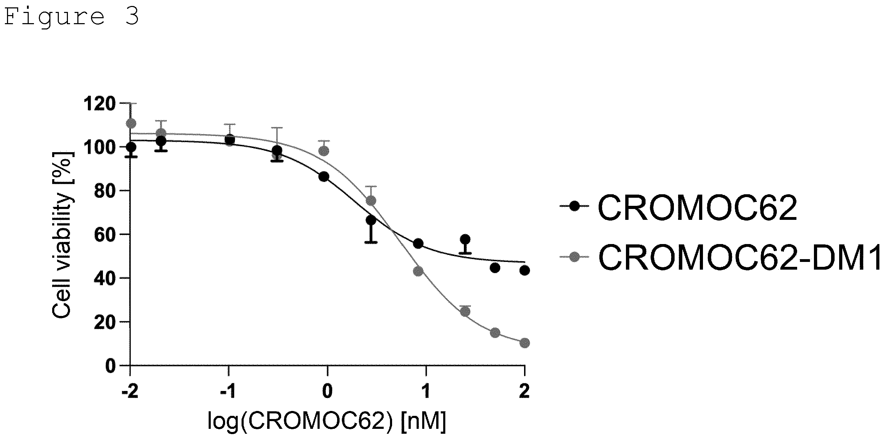

The in vitro activity of CROMOC62 and CROMOC62-DM1 nuclease drug conjugates in the lung adenocarcinoma A549. Dose-response curves were assessed with adenocarcinoma A549 tumour cells. The values obtained from an ATP assay as described in Example 5. Are shown in Figure 3. Whereas the dose response curve of CROMOC62 shows a cytostatic effect, the drug conjugate exhibits cytotoxic activity as shown in Figure 3. Whereas the cell viability of CROMOC62-DM1 is reduced below 5% with increasing dosing, CROMOC62 non-conjugated shows a plateau at 45%. This demonstrates that the conjugation of CROMOC62-DM1 brings about a strong increase in anti-tumour activity.

Example 4

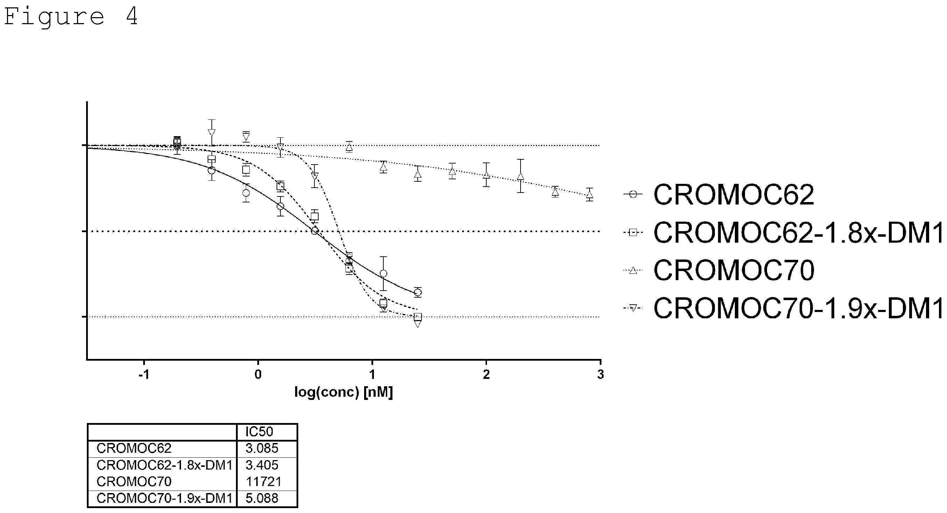

In vitro activity of CROMOC62, CROMOC62-DM1, CROMOC70 and CROMOC70-DM1 nuclease drug conjugates in osteosarcoma U2OS cells. Dose-response curves in U2OS tumour cells and IC50 values obtained from an SRB assay was done as described below and are summarized in Figure 4. With the U2OS cell-line the CROMOC62, CROMOC62-DM1 show similar activity in vitro with IC50 values in the range of 3.1nM and 3.4nM respectively. But in contrast CROMOC70 a nuclease attenuated CROMOC62 variant due to two single point mutations in the nuclease, shows no measurable activity and the corresponding CROMOC70-DM1 conjugate shows an IC50 of 5.1nM reflecting the activity of the conjugated DM1. This example, and comparison to Example 2, 3, 8 shows the advantageous versatility of a double-activity profile were cell-line dependency is compensated, depending on the active-principle which is differential active, most likely due to the genetic background of a tumour. In more detail, i.e., if a tumour cell is DNA double-strand repair deficient then it is shown, that the CROMOC compound shows strong activity and this is enhanced by a toxin conjugated that is capable to enhance this effect, in the case of DM1 a microtubule inhibitor which terminates cells in the G2/M phase of the cell cycle. It is also possible that in a changing tumour population, one principle gets attenuated but the second is still active. The latter is an advantage for an upcoming drug resistant phenomenon during a treatment. Having colocalization of the two activities, due to the covalent conjugate is certainly of advantage compared to combination therapies.

Example 5

In vitro activity of CROMOC62 and CROMOC70-DM1 nuclease drug conjugates in triple negative breast tumours.

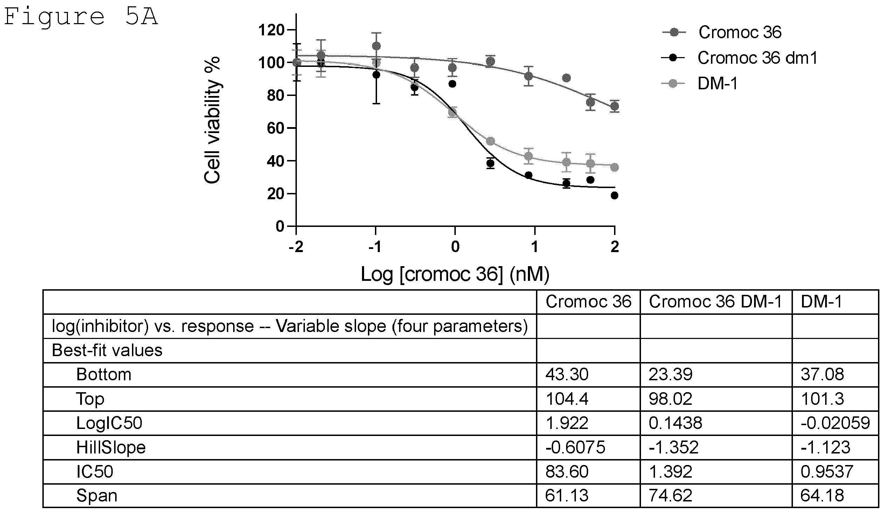

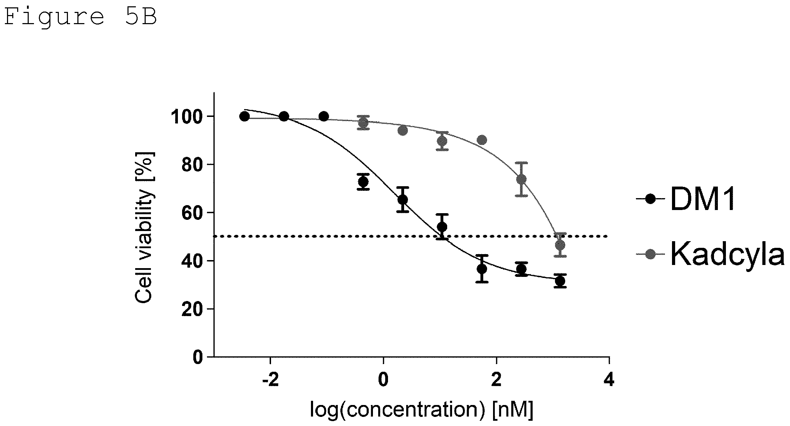

Dose-response curves in triple negative breast tumour cells MDA-MB231 and IC50 values obtained from an ATP assay after a 72h treatment with CROMOC36, CROMOC36-DM1 and the unconjugated DM1 molecule is shown in Figure 5A. The x-axis indicates the drug concentration (nM) in log scale, and the y-axis indicates the % of cell viability as measured by the ATP levels in the treated cells relative to the DMSO control. Whereas the CROMOC36 shows a moderate response of an IC50 of 83.6 nM, the CROMOC36-DM1(DPR 1.3, same molecules as described in Example 6 were used) showed an IC50 of 1.4nM and the free, unconjugated DM1 Toxin showed an IC50 in the range of 0.95nM. This demonstrates the strong response for the nuclease drug conjugate CROMOC36-DM1. Moreover, comparison with the non-conjugated drug demonstrates a efficient uptake as DM1 compares considering a DPR of 1.3 for the conjugated drug a marginal stronger effect of less than two fold. The efficacy in triple negative breast cancer cell lines including Her-2 negative, is further demonstrated as the ADC Trastuzumab-Emtansin (T-DM1, Kadcyla), used for HER2-positive metastatic breast cancer (mBC) in patients who have been treated previously with trastuzumab and paclitaxel or docetaxel as shown in Figure 5B.

ATP assay

The cytotoxic effect of CROMOC-DM1 conjugates was evaluated on the MDA-MB-231 cell line by means of the ATPlite Luminescence ATP Detection Assay System. The cytotoxic effect of the different CROMOC conjugated with DM-1 was compared to that of non-conjugated CROMOCs. Cells treated only with the medium were used as control. Briefly, the cells were seeded in black 96-well plates at a density of 2000 cells/well. After 24 h, cells were treated with increasing concentrations of the conjugates (range 0.02 - 500 nM in CROMOC, n=11) for 72 hours. To quantify ATP production, cells were treated following the manufacturer's instructions, and luminescence was quantified by means of a Victor Nivo multi-plate reader (Perkin Elmer, Waltham, MA, USA). The percentage of cell viability was determined as (luminescence$_{treated\ cells}$/luminescence$_{control\ cells}$) $\times$ 100. IC$_{50}$ values were calculated using GraphPad Prism 9.0 (San Diego, CA, USA) by a nonlinear regression model.

Example 6

Xenograft of a subcutaneous implanted lung adenocarcinoma treated with a CROMOC62-DM1 drug conjugate.

For the analysis of the *in vivo* activity of CROMOC36 drug conjugate compounds, the DM1 conjugates of CROMOC36, CROMOC 1.3 DM1 (CROMOC36-SMCC-DM1conjugate, 1.3 drug to protein ratio (DPR))and CROMOC 3.4 DM1 (CROMOC36-SMCC-DM1conjugate 3.4 DPR)were compared to non-conjugated CROMOC (CROMOC36) on a lung adenocarcinoma xenograft cancer tumour model A549 in athymic nude mice. The tested compounds CROMOC, CROMOC 1.3 DM1, and CROMOC 3.4 DM1 were prepared as described in Example 1, but instead of CROMOC62, which is PEGylated with a 30kDa PEG and maleimide chemistry at the single chain cysteine, CROMOC36 protein was used, which in opposite to CROMOC62 does not contain a cysteine and is PEGylated at the N-terminus using a 30kDa PEG and propionyl aldehyde and tertiary amine coupling. Before the start of the CROMOC treatment, human lung cancer cells (A549 cell line) were inoculated subcutaneously in the flank of each athymic nude mouse. When the tumours had a suitable size (average of 101,48 mm$^3$, day 15), treatments were initiated (Day 15). Vehicle PBS was used as a control for tumour growths. Three intravenous treatments were done 14 days apart on the days 15, 28, 42, and on day 70 response rate was evaluated.

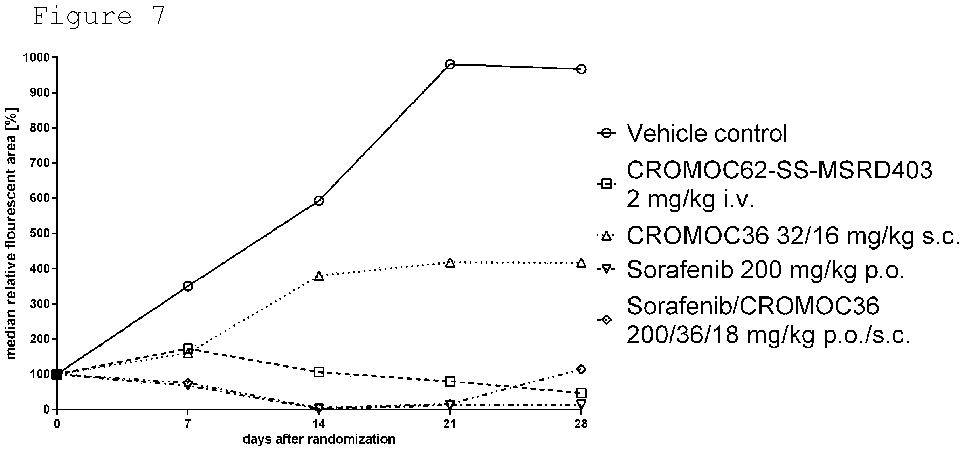

The results are summarized in Figure 7.

For rating of the results RECIST criteria were adapted for mouse xenograft studies. As such, the relative tumour load of an individual tumour on day X ($RTL_x$) is calculated by dividing the absolute tumour volume [in mm$^3$] of the respective tumour on day X ($T_x$) by the absolute tumour load of the same tumour on the day of randomization, i.e., on day 0 ($T_0$), multiplied by 100, as shown by the following equation:

$$RTL_x \ [\%] = \frac{T_x}{T_0} \ x100$$

RTLs are used for growth characterization and compound activity rating as follows:

| Rating | | $RTL_X$ [%] |
|--------|------|------|
| CR | Complete remission | $\leq 10$ |
| PR | Partial remission | $> 10; \leq 50$ |
| MR | Minor remission | $> 50; \leq 75$ |

Groups evaluated out of 10 animals treated were as follows:

| CROMOC | | | 16mg/kg | 1 CR |
|--------|-----|-----|---------|------|
| CROMOC | 1.3 | DM1 | 1mg/kg | 1 CR, 2 MR, |
| CROMOC | 1.3 | DM1 | 2mg/kg | 1 CR, 3 PR, 1MR |
| CROMOC | 3.4 | DM1 | 1mg/kg | 3 CR, 2 MR, |
| CROMOC | 3.4 | DM1 | 2mg/kg | 1 CR, 2 MR |

This Example shows a very strong Tumour Growth inhibition at very low toxicity, and a very relaxed schedule of 14 days at low concentrations of CROMOC as well as DM1. The final macroscopic examination of major organs did not reveal abnormalities in treated groups.

Example 7

Xenograft of hepatocellular carcinoma xenograft orthotopically implanted into liver and treated with a CROMOC62-SS-MSRD403 drug conjugate.

To study the in vivo anti-tumour efficacies of a CROMOC-62-Tubulysine (CROMOC62-SS-MSRD403) drug conjugate, CROMOC62 and CROMOC62-SS-MSRD403 were assessed with sorafenib as a standard in the cell line derived tumour xenograft LIXF HEP3B (hepatocellular carcinoma) implanted orthotopically passaged in NMRI nude mice. The experiment comprised a reference group (Group 1) dosed with phosphate buffered saline and four treatment groups (Groups 2 to 5). Mice received either CROMOC62-SS-MSRD403, CROMOC62 or sorafenib in one appropriate dose level for four weeks or the combination of CROMOC62 and sorafenib in a modulated treatment regimen compared to the corresponding monotherapies. CROMOC62-SS-MSRD403 was given intravenously every second week for 4 weeks at 2 mg/kg/d. CROMOC62 was given subcutaneously every second week for 4 weeks at 32 (induction dosing on day 0) and 16 (day 14 and 28) mg/kg/d. Sorafenib was applied daily for 6 days in mono and combined therapy and additional 6 days (9-14) exclusively in monotherapy per oral at 200 mg/kg/d. The daily dose was split, the daily dosing interval was 6 hours. Mice were either sacrificed individually when their general condition required sacrifice or at termination of the experiment. The group size at the start of dosing was seven to eight mice per group, bearing orthotopic growing LIXF HEP3B. CROMOC62-SS-MSRD403 was applied at one dose level of 2 mg/kg/d. CROMOC62 was given at one dose levels (32 mg/kg on day 0 and 16 mg/kg/d on days 14 and 28) in monotherapy or in combination with sorafenib which was given at one dose level (200 mg/kg/d on days 0-5). The effect of sorafenib monotherapy was also assessed (200 mg/kg/d on day 0-5 and 9-14).

CROMOC62-SS-MSRD403 given at 2 mg/kg in monotherapy presented excellent, statistically significant, anti-tumour activity: CROMOC62-SS-MSRD403 induced three complete remissions, two partial and two minor remissions. CROMOC62 applied at a dose level of 32 mg/kg loading and 16 mg/kg maintenance dose displayed moderate anti-tumour activity. At this dose level, CROMOC62 alone induced two minor remissions and one no change. In combination with sorafenib five complete and two partial remissions were noted. Monotherapy with sorafenib induced as well five complete and two partial remissions. These effects were also statistically significant. A direct comparison of the combination group (CROMOC62 & sorafenib) with sorafenib monotherapy was not possible due to the different treatment schedules for sorafenib. CROMOC62 in combination with sorafenib induced five complete and one minor remission. Thus, treatment displayed excellent antitumoural activity. CROMOC62-SS-MSRD403 and Sorafenib induced equal antitumoural effects. Differences in T/C values were not statistically significant. Metastases could be determined exclusively in the control group in three out of 7 mice from day 28 on (two bone marrow, one lymph node metastases). No metastases were observed in the treatment groups.

Remarkable group median body weight losses were recorded in all treatment groups except CROMOC62-SS-MSRD403, mortalities exclusively in groups treated with sorafenib and/or CROMOC62. CROMOC62-SS-MSRD403 induced no mortalities. CROMOC62 in monotherapy induced one mortality and a body weight loss of 12.2% on day 10. In addition, mice had severe inflammations of the skin at the injection site. One out of eight sorafenib treated mice died during treatment and schedule had to be adapted to the bad overall condition of the treated mice. Nevertheless, the total dose of 12x200 mg/kg/d could be applied. The maximum body weight loss occurred on day 16 and accounted for 2.5%. Combined treatment of sorafenib & CROMOC62 induced two mortalities and a maximum body weight loss of 20.9% on day 21. The above-mentioned inflammations of the skin were also observed in the combination group.

Thus, CROMOC62-SS-MSRD403 showed very promising antitumoural activity in orthotopic implanted liver carcinoma LIXF HEP3B exceeding the antitumoural activity of sorafenib by improving the safety concerns of the antitumoural treatment. Given the fact, that a very long treatment cycle of 14 days was applied a reduction to 7 days is considered the optimal schedule, a broad pharmacological window can be expected.

Example 8

Sensitivity of cell lines to CROMOC62, and the Tubulysine MSRD403 nuclease drug conjugates CROMOC62-SS-MSRD403 and the endonuclease impaired version CROMOC70-SS-MSRD403. IC50 values were assessed in various tumour cell-lines using an SRB assay. Treatment of the cell-lines was for 72 hours with the indicated compounds synthesized as described in Example 2. The tumour indications and representative tumour cell-lines tested were, bladder: UMUC3, T24, J82, EJ28, CLS439, 5637,bone: U2OS, SAOS2, RDES, MHHES1, MG63, brain: U87MG, SNB75, SKNSH, SKNAS, SF295, SF268, breast: SKBR3, MT3, MDAMB468, MDAMB436, MDAMB231, MCF7, JIMT1, HS578T, BT20, cervix: HELA, CASKI, C33A, colon: SW620, LOVO, HT29, HCT15, HCT116, DLD1, COLO678, COLO205, CACO2, connective-tissue: HT1080, kidney: UO31, HEK293, CAKI1, ACHN, 786O, liver: SKHEP1, PLCPRF5, HEPG2, lung: NCIH82, NCIH460, NCIH358M, NCIH292, IMR90, CALU6, A549, muscle: TE671, RD, HS729, A673, A204, ovary: SKOV3, OVCAR4, OVCAR3, IGROV1, EFO21, A2780, pancreas: PANC1005, PANC1, MIAPACA2, BXPC3, ASPC1, placenta: JEG3, JAR, prostate: PC3, DU145, 22RV1,skin: SKMEL5, SKMEL28, MDAMB435, A431,uterus: SKLMS1, NON tumour cells: freshly obtained PBMC. For rating of the pan-tumour activity of the compounds the results were summarized into percentage of cells that exhibit an IC50 in either range of <10nM, <100nM and ≥ 100nM. This allowed an overall rating of anti-tumour activity of the compounds as follows:

| | |
|---|---|
| CROMOC62 shows | 32% <10nM, 19% <100nM, and 49% >100 nM |
| CROMOC62-SS-MSRD403 | 48% <10nM, 48% <100nM, and 4% >100 nM |
| CROMOC70-SS-MSRD403 | 37% <10nM, 58% <100nM, and 5% >100 nM |

The non-tumour cells PBMC showed with all compounds IC50 values in the $\mu$M range beyond the 10$\mu$M detection maximum of the assay.

The results show that the combination of two active principles, a DNA double strand hydrolyzing activity and a microtubule inhibitor, as is the case for Nuclease Drug candidates, significantly brings about lower IC50 values as compared to the single active principles tested using an ample cell panel, and these results do not hold for non-tumour PBMC cells.

SRB assay was done as follows:

Monolayer Assay using sulphorhodamine B (SRB) staining.

Cells were harvested from exponential phase cultures and depending on the cell line 10.000 to 30.000 cells were plated in 96-well flat-bottom microtiter plates. After a 24 h recovery period to allow cells to resume exponential growth, supernatant was discarded and 100 $\mu$l of culture medium (eight control wells/plate) or culture medium with the test compound were added by a liquid handling robotic system (Microlab $^{\circledR}$ Starlet, Hamilton) and treatment continued for 72 hrs. Compounds were applied at 9 concentrations eight wells/concentration). At least three independent experiments were performed. For SRB staining, cells were fixed with trichloroacetic acid (TCA) 50% w/v in dH$_2$O for 1h at 4 °C (final concentration of TCA=10%) washed with a plate washer (Hydroflex, Tecan$^{\circledcopy}$) for five times with acetic acid 1% in dH$_2$O, and stained with 100$\mu$l of staining solution containing sulphorhodamine B (SRB) 0.4% w/v in acetic acid 1% in dH$_2$O at room temperature for 10min following 4 washes in acetic acid 1% in dH$_2$O and finally 100$\mu$l of developer solution tris base 10mM for 5 minutes with shaking. Optical density was assessed using a plate-reader (Sunrise, Tecan$^{\circledcopy}$) at 540 nm. Concentrations required for 50% inhibition of cell growth (IC$_{50}$) were calculated by non-linear regression (log[conc. of inhibitor] versus response (% T/C)) using the analysis software GraphPad Prism$^{\circledR}$ , Prism 5 for windows, version 5.01 (GraphPad Software Inc., CA).

**Claims**

1. Compound comprising or consisting of

    (i) a polypeptide with the general formula (I):

$$X_0GX_1X_2GX_3X_4X_5GX_6X_7X_8GX_9X_{10}X_{11}X_{12}X_{13}X_{14},$$

    wherein

    G is glycine;
    X$_0$ is present or not and, if present, is an amino acid linker;
    X$_1$ is N or S, wherein N is asparagine and S is serine;
    X$_2$ is S or T, wherein S is serine and T is threonine;
    X$_3$ is present or not and, if present, is S, wherein S is serine;
    X$_4$ is present or not and, if present, is G, wherein G is glycine;
    X$_5$ is a basic polypeptide stretch consisting of 5 basic amino acid residues selected from R and K, wherein R is arginine and K is lysine, preferably wherein X$_5$ comprises at least 3 K amino acid residues, especially wherein X$_5$ is KKKKK or KRKKK;
    X$_6$ is a basic amino acid residue selected from R and K, wherein R is arginine and K is lysine, preferably wherein X$_6$ is K;
    X$_7$ is S or L, wherein S is serine and L is leucine;
    X$_8$ is present or not and, if present, is a polypeptide stretch consisting of the amino acid sequence GLGS, wherein G is glycine, L is leucine and S is serine;
    X$_9$ is a basic polypeptide stretch consisting of 3 basic amino acid residues selected from R and K, wherein R is arginine and K is lysine, preferably wherein X$_9$ comprises at least 2 K amino acid residues, especially wherein X$_9$ is KKK or KKR;
    X$_{10}$ is present or not and, if present, is L or a polypeptide stretch consisting of the amino acid sequence DPL or DPC, wherein L is leucine, D is aspartic acid, P is proline, and C is cysteine;
    X$_{11}$ is present or not and, if present, is a polypeptide stretch consisting of the amino acid sequence LR or GSGL, wherein L is leucine, R is arginine, G is glycine, and S is serine;

$X_{12}$ is present or not and, if present, is a basic polypeptide stretch with at least 20 % basic amino acid residues selected from K and R and at least a P residue, preferably wherein $X_{12}$ is selected from KYKPKL, KYKPKLGT, or $GX_3X_4X_5GX_6X_7X_8GX_9X_{10}$, wherein K, R, P, L, G, T, $X_3$, $X_4$, $X_5$, $GX_6$, $X_7$, $X_8$, $GX_9$, and $X_{10}$, are defined as above and wherein Y is tyrosine;

$X_{13}$ is present or not and, if present, is a polypeptide stretch consisting of the amino acid sequences GS, GST, GST, GSG, GSTG, or GSGL, wherein G, S, T and L are defined as above;

$X_{14}$ is present or not and, if present, is an amino acid linker;

wherein the polypeptide has a length from 20 to 75 amino acid residues, preferably from 24 to 65 amino acid residues, especially from 25 to 60 amino acid residues; and

(ii) at least one payload molecule to be delivered into a biological cell,

wherein (i) and (ii) are covalently linked to each other, and

wherein (ii) is a single chain restriction enzyme and wherein the compound further comprises a toxin as second (ii) covalently coupled to (i), preferably in the order (ii) single chain restriction enzyme - (i) - (ii) toxin, wherein the toxin is selected from microtubule inhibitors, especially auristatin, maytansinoids, tubulysins; cytotoxic small molecules with a molecular mass of 300 to 1300 Daltons, Dimethyl-SGD-1882,CAS1222490-34-7, SN-38, 7-Ethyl-10-hydroxycamptothecin, CAS86639-52-3, Duocarmycin, CAS118292-34-5, Tubulysin-beta, Cas 1943604-24-7, Tubulysin A(TubA) CAS 205304-86-5, Tubulysin, Tubulysin B, M, IM1, IM2, IM3, CAS205304-86-5, Tubulysin C, Tubulysin D, Tubulysin E, Tubulysin F, Tubulysin G, Tubulysin H, Tubulysin I, Maytansinoid AP-3 CAS 66547-09-Auristatin E CAS 160800-57-7, Auristatin F CAS163768-50-1, Dolastatin 10 CAS10417-88-4, Maytansinol CAS57103-68-1, Monomethyl auristatin D (MMAD) CAS203849-91-6, Monomethylauristatin F(MMAF) CAS745017-94-1, Monomethyl-lauristatin E (MMAE) CAS474645-27-7, DM1 Maytansinoid CAS139504-50-0, DM4 Maytansinoid CAS799840-96-3, Maytansine DM3 CAS 796073-54-6, Pyrrolobenzodiazepine (PBDs), Rebeccamycin Sandramycin CAS100940-65-6,10-Deacetyl-7-xylosyl paclitaxel CAS90332-63-1, alpha-Amanitin CAS23109-05-9, gamma-Amanitin, Ansamitocin P-3 CAS66584-72-3, and Calicheamicin CAS108212-75-5.

2. Compound according to claim 1, wherein $X_1$ is N, $X_2$ is S, $X_3$ is S, $X_4$ is G, $X_5$ is KKKKK or KRKKK, $X_6$ is K, $X_9$ is KKK or KKR, and $X_{10}$ is L or a polypeptide stretch consisting of the amino acid sequence DPL or DPC, wherein N, S, G, R, L, D, P and C are defined as above.

3. Compound according to claim 1 or 2, wherein (ii) is selected from the group

GNSGSGKRKKKGKGLGKKRDPCLRKYKPKLGTGSTG(HHHHHH) (DDM11),
GNSGKRKKKGKGSGLGSGKKRDPCLRKYKPKLGSTG(HHHHHH) (DDM18),
GNSGSGKRKKKGKGLGKKRDPCLRKYKPKLGTGSTG(HHHHHH) (DDM21),
GNSGSGKRKKKGKGSGLGSGKKRDPLGSTG(HHHHHH) (DDM26),
GNSG(HHHHHH)GSTGGKRKKKGKGSGLGSGKKRDPL (DDM27),
GNSGSGKRKKKGKGSGLGSGKKKDPLGSTG(HHHHHH) (DDM28),
GNSG(HHHHHH)GSTGGKRKKKGKGSGLGSGKKKDPL (DDM29),
GNSGSGKKKKKGKGSGLGSGKKRDPLGSTG(HHHHHH) (DDM30),
GNSG(HHHHHH)GSTGGKKKKKGKGSGLGSGKKRDPL (DDM31),
GNSGSGKKKKKGKGSGLGSGKKKDPLGSTG(HHHHHH) (DDM32),
GNSG(HHHHHH)GSTGGKKKKKGKGSGLGSGKKKDPL (DDM33),
GNSGSGKKKKKGKGSGLGSGKKKDPL (DDM34),
GNSGSGKKKKKGKGSGLGSGKKKLGSTG(HHHHHH) (DDM35),
GNSGSGKKKKKGKGSGLGSGKKKDPLGSGLGSGKKKKKGKGSGLGSGKKKDPLGST (DDM36), and
GNSGSGKKKKKGKGSGLGSGKKKDPLGSGLGSGKKKKKGKGSGLGSGKKKDPLGSTG(HHHHHH　) GST (DDM44),
wherein (HHHHHH; a histidine tag) may be present or not; preferably
GNSGSGKRKKKGKGLGKKRDPCLRKYKPKLGTGSTG (DDM11),
GNSGKRKKKGKGSGLGSGKKRDPCLRKYKPKLGSTG (DDM18),
GNSGSGKRKKKGKGLGKKRDPCLRKYKPKLGTGSTG (DDM21),
GNSGSGKRKKKGKGSGLGSGKKRDPLGSTG (DDM26),
GNSGGSTGGKRKKKGKGSGLGSGKKRDPL (DDM27),
GNSGSGKRKKKGKGSGLGSGKKRDPLGSTG (DDM28),

GNSGGSTGGKRKKKGKGSGLGSGKKKDPL (DDM29),
GNSGSGKKKKKGKGSGLGSGKKRDPLGSTG (DDM30),
GNSGGSTGGKKKKKGKGSGLGSGKKRDPL (DDM31),
GNSGSGKKKKKGKGSGLGSGKKKDPLGSTG (DDM32),
GNSGSGKKKKKGKGSGLGSGKKKDPL (DDM34),
GNSGSGKKKKKGKGSGLGSGKKKDPLGSGLGSGKKKKKGKGSGLGSGKKKDPLGST (DDM36),
and GNSGSGKKKKKGKGSGLGSGKKKDPLGSGLGSGKKKKKGKGSGLGSGKKKDPLGSTGGST (DDM44),
especially
GNSGSGKKKKKGKGSGLGSGKKKDPLGSGLGSGKKKKKGKGSGLGSGKKKDPLGST (DDM36),
and GNSGSGKKKKKGKGSGLGSGKKKDPLGSGLGSGKKKKKGKGSGLGSGKKKDPLGSTGGST (DDM44).

4. Compound according to any one of claims 1 to 3, wherein (ii) is a chemotherapeutic molecule, a cytotoxic molecule, a DNA damaging molecule, an anti-metabolite molecule, a therapeutic molecule, a small molecule with therapeutic effect inside a biological cell, a DNA molecule, an RNA molecule, an antibody molecule or an antibody derivative having an antibody-like function, a restriction endonuclease, a nicking enzyme, or DNA- or RNA-dependent endonucleases which show double strand breaking nuclease double strand/single strand breaking activity or no nuclease activity, more preferred wherein the payload molecule is a class II restriction endonuclease, such as PvuII, EcoRV, PvuII, HinfI, or a sc homodimer, a subunit or a functional fragment thereof; especially wherein the payload molecule is selected from the group

microtubule inhibitors, especially auristatin, maytansinoids, tubulysins; cytotoxic small molecules with a molecular mass of 300 to 1300 Daltons, Dimethyl-SGD-1882,CAS1222490-34-7, SN-38, 7-Ethyl-10-hydroxycamptothecin, CAS86639-52-3, Duocarmycin, CAS118292-34-5, Tubulysin-beta, Cas 1943604-24-7, Tubulysin A(TubA) CAS 205304-86-5, Tubulysin, Tubulysin B, M, IM1, IM2, IM3, CAS205304-86-5, Tubulysin C, Tubulysin D, Tubulysin E, Tubulysin F, Tubulysin G, Tubulysin H, Tubulysin I, Maytansinoid AP-3 CAS 66547-09-Auristatin E CAS 160800-57-7, Auristatin F CAS163768-50-1, Dolastatin 10 CAS10417-88-4, Maytansinol CAS57103-68-1, Monomethyl auristatin D (MMAD) CAS203849-91-6, Monomethylauristatin F(MMAF) CAS745017-94-1, Monomethylauristatin E (MMAE) CAS474645-27-7, DM1 Maytansinoid CAS139504-50-0, DM4 Maytansinoid CAS799840-96-3, Maytansine DM3 CAS 796073-54-6, Pyrrolobenzodiazepine (PBDs), Rebeccamycin Sandramycin CAS100940-65-6,10-Deacetyl-7-xylosyl paclitaxel CAS90332-63-1, alpha-Amanitin CAS23109-05-9, gamma-Amanitin, Ansamitocin P-3 CAS66584-72-3, Calicheamicin CAS108212-75-5,

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIY,

SHPDLNKLLELWPHIQEYQDLALKHGINDIFQDNGGKLLQVLLITGLTVLPGREGNDAVDNAGQ
EYELASINIDLTKGFSTHHHMNPVIIAKYRQVPWIFAIYRGIAIEAIYRLEPKD-
LEFYYDKWERKWYSDGHKDINNPKIPVKYVMEHGTKIY,

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGGSGGSSHPDLNKLLELWPHIQE
YQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERK
WYSDGHKDINNPKIPVKYVMEHGTKIY,

SHPDLNKLLELWPHIQEYQDLALKHGINDIFQDNGGKLLQVLLITGLTVLPGREGNDAVDNAGQ
EYELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGGSGGSSHPDLNKLLELWPHIQE
YQDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERK
WYSDGHKDINNPKIPVKYVMEHGTKIY,

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCSGGSSHPDLNKLLELWPHIQE
YQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERK
WYSDGHKDINNPKIPVKYVMEHGTKIY,

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCSGGSSHPDLNKLLELWPHIQ
EYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERK
WYSDGHKDINNPKIPVKYVMEHGTKIY,

SHPDLNKLLELWPHIQEYQDLALKHGINDIFQDNGGKLLQVLLITGLTVLPGREGNDAVDNAGQ
EYELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCSGGSSHPDLNKLLELWPHIQE
YQDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERK
WYSDGHKDINNPKIPVKYVMEHGTKIY,

SHPDLNKLLELWPHIQEYQDLALKHGINDIFQDNGGKLLQVLLITGLTVLPGREGNDAVDNAGQ
EYELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCSGGSSHPDLNKLLELWPHIQ
EYQDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERK
WYSDGHKDINNPKIPVKYVMEHGTKIY,

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCCSGGSSHPDLNKLLELWPHI
QEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERK
WYSDGHKDINNPKIPVKYVMEHGTKIY,

SHPDLNKLLELWPHIQEYQDLALKHGINDIFQDNGGKLLQVLLITGLTVLPGREGNDAVDNAGQ
EYELASINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCCCSGGSSHPDLNKLLELWPHI
QEYQDLALKHGINDIFQDNGGKLLQVLLITGLTVLPGREGNDAVDNAGQE-
YELASINIDLTKGFSTHHHMNPVIIAKYRQVPWIFAIYRGIAIEAIYRLEPKDLEFYYDKWERK
WYSDGHKDINNPKIPVKYVMEHGTKIY,

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIY**GSGGCAACSGGS**SHPDLNKLLELWPH
IQEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERK
WYSDGHKDINNPKIPVKYVMEHGTKIYGNSGSGKKKKKGKGSGLG-
SGKKKDPLGSGLGSGKKKKKGKGSGLGSGKKKDPLGST,

and

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIY**GSGCAACAACSGS**SHPDLNKLLELWP
HIQEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERK
WYSDGHKDINNPKIPVKYVMEHGTKIYGNSGSGKKKKKGKGSGLG-
SGKKKDPLGSGLGSGKKKKKGKGSGLGSGKKKDPLGST.

5. Compound according to any one of claims 1 to 4, wherein the compound additionally comprises a homopolymer moiety covalently attached to the polypeptide with the general formula (I) or with the payload molecule (ii), preferably wherein the homopolymer is selected from the group of polyethylene glycol (PEG), especially a PEG with a MW of 5 to 30 kDa; dextran, polysialic acids, hyaluronic acid, dextrin, hydroxyethyl starch, or poly(2-ethyl 2-oxazoline.

6. Compound according to any one of claims 1 to 5, wherein the compound additionally comprises a restriction endonuclease as a payload molecule to be delivered into a biological cell covalently attached to the polypeptide with the general formula (I), preferably a class II restriction endonuclease, especially a PvuII restriction endonuclease or a derivative thereof wherein the derivative is a single chain PvuII restriction endonuclease, such as the derivative comprising the amino acid sequence

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIY,

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGG,

SGGSSHPDLNKLLELWPHIQEYQDLALKHGINDIFQDNGGKLLQVLLITGLTVLPGREGNDAVD
NAGQEYELKSINIDLTKGFSTHHHMNPVIIAKYRQVPWIFAIYRGIAIEAIYRLEPKD-
LEFYYDKWERKWYSDGHKDINNPKIPVKYVMEHGTKIY,

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGNSG,

MSHPDLNKLLELWPHIQEYQDLALKHGINDIFQDNGGKLLQVLLITGLTVLPGREGNDAVDNAG
QEYELKSINIDLTKGFSTHHHMNPVIIAKYRQVPWIFAIYRGIAIEAIYRLEPKD-
LEFYYDKWERKWYSDGHKDINNPKIPVKYVMEHGTKIY,

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGC,

CSGGSSHPDLNKLLELWPHIQEYQDLALKHGINDIFQDNGGKLLQVLLITGLTVLPGREGNDAV
DNAGQEYELKSINIDLTKGFSTHHHMNPVIIAKYRQVPWIFAIYRGIAIEAIYRLEPKD-
LEFYYDKWERKWYSDGHKDINNPKIPVKYVMEHGTKIY,

MSHPDLNKLLELWPHIQEYQDLALKHGINDIFQDNGGKLLQVLLITGLTVLPGREGNDAVDNAG
QEYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGCSGGSSHPDLNKLLELWPHIQE
YQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERK
WYSDGHKDINNPKIPVKYVMEHGTKIY,

MSHPDLNKLLELWPHIQEYQDLALKHGINDIFQDNGGKLLQVLLITGLTVLPGREGNDAVDNAG
QEYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGSGGSSHPDLNKLLELWPHIQEY
QDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERK
WYSDGHKDINNPKIPVKYVMEHGTKIY,

MSHPDLNKLLELWPHIQEYQDLALKHGINDIFQDNGGKLLQVLLITGLTVLPGREGNDAVDNAG
QEYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGSGGS,

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIYGSGGSGGS,

GSGGSGGSSHPDLNKLLELWPHIQEYQDLALKHGINDIFQDNGGKLLQVLLITGLTVLPGREGN
DAVDNAGQEYELKSINIDLTKGFSTHHHMNPVIIA-
KYRQVPWIFAIYRGIAIEAIYRLEPKDLEFYYDKWERKWYSDGHKDINNPKIPVKYVMEHGTKI
Y,

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIY**GSGGCAACSGGS**SHPDLNKLLELWPH
IQEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERK
WYSDGHKDINNPKIPVKYVMEHGTKIYGNSGSGKKKKKGKGSGLG-
SGKKKDPLGSGLGSGKKKKKGKGSGLGSGKKKDPLGST,

or

SHPDLNKLLELWPHIQEYQDLALKHGINDIFQDNGGKLLQVLLITGLTVLPGREGNDAVDNAGQ
EYELKSINIDLTKGFSTHHHMNPVIIAKYRQVPWIFAIYRGIAIEAIYRLEPKD-
LEFYYDKWERKWYSDGHKDINNPKIPVKYVMEHGTKIY**GSGCAACAACSGS**SHPDLNKLLELWP
HIQEYQDLALKHGINDIFQDNGGKLLQVLLITGLTVLPGREGNDAVDNAGQE-
YELKSINIDLTKGFSTHHHMNPVIIAKYRQVPWIFAIYRGIAIEAIYRLEPKDLEFYYDKWERK
WYSDGHKDINNPKIPVKYVMEHGTKIYGNSGSGKKKKKGKGSGLG-
SGKKKDPLGSGLGSGKKKKKGKGSGLGSGKKKDPLGST.

7. Compound according to any one of claims 1 to 6, wherein the polypeptide with the general formula (I) is covalently linked to another moiety by a linker, which is a cleavable or a non-cleavable linker, wherein the another moiety is preferably a payload molecule to be delivered into a biological cell, a labelling group, and/or a homopolymer.

8. Compound according to any one of claims 1 to 7, wherein the compound further comprises a capping group, preferably an alkoxy, especially a methoxy, ethoxy, propoxy, or butoxy group; a halogen atom; or a tosylate; isocyanate, hydrazine hydrate, maleimide, orthopyridyl disulfide, N-succinimidyloxy, sulfo-N-succinimidyloxy, 1-benzotriazol, 1-imidazolyloxy, p-nitrophenyloxy, or aldehyde.

9. Compound according to any one of claims 1 to 8, wherein the compound further comprises a linker, wherein the linker is an amino acid linker of 1 to 20 amino acid residues in length, preferably from 1 to 15 amino acid residues, more preferred from 7 to 13 amino acid residues, especially of 8 to 12 amino acid residues.

10. Compound according to any one of claims 1 to 9, wherein the compound further comprises a linker, wherein the linker is an amino acid linker, selected from a linker comprising or consisting of cysteine, serine or glycine residues, preferably a single or two adjacent cysteine residue(s); an amino acid sequence comprising glycine and serine residues or comprising or consisting of the amino acid sequences GSG, SGG, GGC, GCS, CSG, GGS, GSGG, SGGS, GSGGC, CSGGS, GSGGSGGS, GSGGCSGGS, GSGGCCCSGGS, GSGGCSGGS, GSGGCAACSGGS or GSGCAACAACSGS.

11. Compound according to any one of claims 1 to 10, wherein the compound further comprises as payload two monomeric subunits or parts thereof of a type II restriction endonuclease, preferably, two monomeric alpha-helical subunits of a type II restriction endonuclease covalently connected by a linker to the polypeptide with the general formula (I); or wherein the compound further comprises as payload two monomeric alpha-helical subunits of PvuII, preferably wherein the two monomeric subunits or parts thereof of the type II restriction endonuclease PvuII are covalently connected by a linker to the polypeptide with the general formula (I).

12. Compound according to any one of claims 1 to 11, wherein the compound is selected from the group GNSGSGKKKKKGKGSGLGSGKKKDPL (DDM34) and GNSGSGKKKKKGKGSGLGSGKKKDPLGSGLGSGKKK KKGKGSGLGSGKKKDPLGST (DDM36).

13. Compound according to any one of claims 1 to 12, wherein the polypeptide with the general formula (I) is covalently coupled to the C-terminus of a payload molecule to be delivered into a biological cell which is a class II restriction endonuclease, preferably PvuII or a subunit thereof, wherein the payload molecule is optionally attached at its C-terminus to a linker molecule comprising 8 to 12 amino acids comprising or consisting of glycine, serine and/or cysteine residues, and wherein the polypeptide with the general formula (I) is covalently coupled to the C-terminus of the payload molecule or the linker molecule, wherein the compound comprises one or two payload molecule(s), optionally separated by the linker molecule comprising 8 to 12 amino acids comprising or consisting of glycine, serine and/or cysteine residues; and/or wherein the polypeptide with the general formula (I) is covalently coupled to the C-terminus of a payload molecule to be delivered into a biological cell, wherein the compound comprises a first payload molecule which is the first subunit of PvuII linked at its C-terminus to a linker molecule comprising 8 to 12 amino acids comprising or consisting of glycine, serine and/or cysteine residues, wherein the linker molecule is attached at its C-terminus to the second subunit of PvuII and wherein the polypeptide with the general formula (I) is covalently coupled to the C-terminus of the second subunit of PvuII.

14. Compound according to any one of claims 1 to 19, for use in the treatment of a tumour patient, preferably wherein the tumour patient is suffering from neuroblastoma, colon carcinoma, hepatocellular carcinoma, Small Cell Lung carcinoma, ovarian carcinoma, lung carcinoma, bladder carcinoma, prostate carcinoma, uterus carcinoma, cervix carcinoma, placental carcinoma, breast carcinoma, kidney carcinoma, liver carcinoma, pancreas carcinoma, muscle carcinoma, skin carcinoma, connective tissue carcinoma, bone carcinoma, and any of these carcinomas wherein the patient has already developed metastases, especially for the treatment of a patient having neuroblastoma, colon carcinoma, hepatocellular (liver) carcinoma, Small Cell Lung carcinoma, lung carcinoma, breast carcinoma, pancreas carcinoma, and any of these carcinomas wherein the patient has already developed metastases.

15. Pharmaceutical preparation comprising a compound according to any one of claims 1 to 13 and a protein kinase inhibitor (PKI), preferably a DNA-dependent PKI, more preferred a Non Homologous End Joining (NHEJ) and V(D)J repair factor DNA-dependent PKI, especially 7-Methyl-2-[(7-methyl[1,2,4]triazolo[1,5-a]pyridin-6-yl)amino]-9-(tetrahydro-2H-pyran-4-yl)-7,9-dihydro-8H-purin-8-one (AZD7648).

Figure 1

# Figure 2A

Figure 2B

Figure 3

Figure 4

| | IC50 |
|---|---|
| CROMOC62 | 3.085 |
| CROMOC62-1.8x-DM1 | 3.405 |
| CROMOC70 | 11721 |
| CROMOC70-1.9x-DM1 | 5.088 |

- CROMOC62
- CROMOC62-1.8x-DM1
- CROMOC70
- CROMOC70-1.9x-DM1

log(conc) [nM]

Figure 5A

| | Cromoc 36 | Cromoc 36 DM-1 | DM-1 |
|---|---|---|---|
| log(inhibitor) vs. response -- Variable slope (four parameters) | | | |
| Best-fit values | | | |
| Bottom | 43.30 | 23.39 | 37.08 |
| Top | 104.4 | 98.02 | 101.3 |
| LogIC50 | 1.922 | 0.1438 | -0.02059 |
| HillSlope | -0.6075 | -1.352 | -1.123 |
| IC50 | 83.60 | 1.392 | 0.9537 |
| Span | 61.13 | 74.62 | 64.18 |

Figure 5B

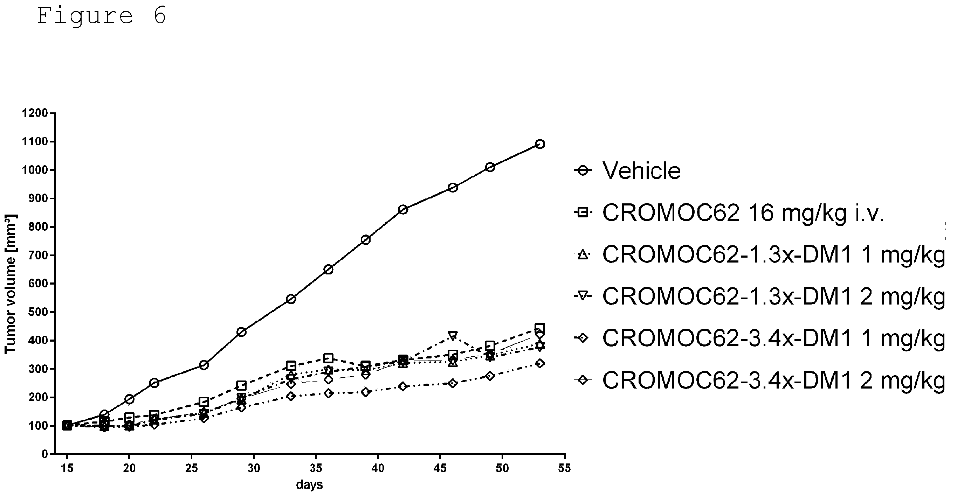

Figure 6

EP 4 480 497 A1

EP 4 480 497 A1

Figure 7

Europäisches
Patentamt

European
Patent Office

Office européen
des brevets

# EUROPEAN SEARCH REPORT

**Application Number**

EP 23 18 0717

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/192879 A1 (UNIV FLORIDA STATE RES FOUND [US]) 15 September 2022 (2022-09-15) * claim 14; sequence 364 * | 1-15 | INV. A61K47/62 A61K47/64 A61K47/68 |
| A | CN 113 332 448 A (ZHEJIANG FONOW MEDICINE CO LTD) 3 September 2021 (2021-09-03) * claim 6; sequence 15 * | 1-15 | A61P35/00 |
| A | CN 108 570 108 A (ZHEJIANG REACHALL PHARMACEUTICAL CO LTD) 25 September 2018 (2018-09-25) * claim 17; sequence 15 * | 1-15 | |
| A,D | WO 2004/092194 A2 (ICGEB [IT]; KUEHNE CHRISTIAN [AT]; SIMONCSITS ANDRAS [IT]) 28 October 2004 (2004-10-28) * claims 1-10 * | 1-15 | |
| A | CATHERINE DE COUPADE ET AL: "Novel human-derived cell-penetrating peptides for specific suncellular delivery of therapeutic biomolecules", BIOCHEMICAL JOURNAL, PUBLISHED BY PORTLAND PRESS ON BEHALF OF THE BIOCHEMICAL SOCIETY, GB, vol. 390, no. part 2, 23 August 2005 (2005-08-23), pages 407-417, XP008126272, ISSN: 0264-6021, DOI: 10.1042/BJ20050401 | 1-15 | TECHNICAL FIELDS SEARCHED (IPC) A61K |
| E | WO 2023/111213 A1 (RDP PHARMA AG [CH]; PAGS CO LTD [KR]) 22 June 2023 (2023-06-22) * claim 1; sequences 1-40 * | 1-15 | |

The present search report has been drawn up for all claims

| Place of search | Date of completion of the search | Examiner |
|---|---|---|
| The Hague | 22 November 2023 | Langer, Miren |

EPO FORM 1503 03.82 (P04C01)

## ANNEX TO THE EUROPEAN SEARCH REPORT
## ON EUROPEAN PATENT APPLICATION NO.

EP 23 18 0717

This annex lists the patent family members relating to the patent documents cited in the above-mentioned European search report.
The members are as contained in the European Patent Office EDP file on
The European Patent Office is in no way liable for these particulars which are merely given for the purpose of information.

22-11-2023

| Patent document cited in search report | | | Publication date | Patent family member(s) | | | Publication date |
|---|---|---|---|---|---|---|---|
| WO 2022192879 | A1 | | 15-09-2022 | US | 2022287968 | A1 | 15-09-2022 |
| | | | | WO | 2022192879 | A1 | 15-09-2022 |
| CN 113332448 | A | | 03-09-2021 | CN | 108570108 | A | 25-09-2018 |
| | | | | CN | 113332447 | A | 03-09-2021 |
| | | | | CN | 113332448 | A | 03-09-2021 |
| | | | | CN | 113336830 | A | 03-09-2021 |
| CN 108570108 | A | | 25-09-2018 | CN | 108570108 | A | 25-09-2018 |
| | | | | CN | 113332447 | A | 03-09-2021 |
| | | | | CN | 113332448 | A | 03-09-2021 |
| | | | | CN | 113336830 | A | 03-09-2021 |
| WO 2004092194 | A2 | | 28-10-2004 | AT | E374826 | T1 | 15-10-2007 |
| | | | | AU | 2004230254 | A1 | 28-10-2004 |
| | | | | CA | 2522525 | A1 | 28-10-2004 |
| | | | | CN | 1768142 | A | 03-05-2006 |
| | | | | DE | 602004009301 | T2 | 17-07-2008 |
| | | | | DK | 1616011 | T3 | 04-02-2008 |
| | | | | EP | 1616011 | A2 | 18-01-2006 |
| | | | | ES | 2293247 | T3 | 16-03-2008 |
| | | | | JP | 4794432 | B2 | 19-10-2011 |
| | | | | JP | 2006523448 | A | 19-10-2006 |
| | | | | JP | 2011182792 | A | 22-09-2011 |
| | | | | KR | 20060003029 | A | 09-01-2006 |
| | | | | KR | 20110074948 | A | 04-07-2011 |
| | | | | PL | 1616011 | T3 | 30-05-2008 |
| | | | | PT | 1616011 | E | 07-01-2008 |
| | | | | US | 2006292629 | A1 | 28-12-2006 |
| | | | | US | 2011033384 | A1 | 10-02-2011 |
| | | | | US | 2015018409 | A1 | 15-01-2015 |
| | | | | WO | 2004092194 | A2 | 28-10-2004 |
| WO 2023111213 | A1 | | 22-06-2023 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2022086215 W
- WO 2004092194 A2
- WO 2020214846 A1
- WO 2004013205 A
- WO 2004005327 A
- WO 2008138561 A1

### Non-patent literature cited in the description

- *CHEMICAL ABSTRACTS*, 1222490-34-7
- *CHEMICAL ABSTRACTS*, 86639-52-3
- *CHEMICAL ABSTRACTS*, 118292-34-5
- *CHEMICAL ABSTRACTS*, 1943604-24-7
- *CHEMICAL ABSTRACTS*, 205304-86-5
- *CHEMICAL ABSTRACTS*, 66547-09
- *CHEMICAL ABSTRACTS*, 160800-57-7
- *CHEMICAL ABSTRACTS*, 163768-50-1
- *CHEMICAL ABSTRACTS*, 10417-88-4
- *CHEMICAL ABSTRACTS*, 57103-68-1
- *CHEMICAL ABSTRACTS*, 203849-91-6
- *CHEMICAL ABSTRACTS*, 745017-94-1
- *CHEMICAL ABSTRACTS*, 474645-27-7
- *CHEMICAL ABSTRACTS*, 139504-50-0
- *CHEMICAL ABSTRACTS*, 799840-96-3
- *CHEMICAL ABSTRACTS*, 796073-54-6
- *CHEMICAL ABSTRACTS*, 100940-65-6
- *CHEMICAL ABSTRACTS*, 90332-63-1
- *CHEMICAL ABSTRACTS*, 23109-05-9
- *CHEMICAL ABSTRACTS*, 66584-72-3
- *CHEMICAL ABSTRACTS*, 108212-75-5
- **PASUT**. *Polymers*, 2014, vol. 6, 160-178
- **GRIGOLETTO et al.** *Nanomed. Nanobiotechnol.*, 2020, e1689
- **PINGOUD et al.** *NAR*, 2001, vol. 29, 3705-3727
- *NAR*, 2014, vol. 42, 7489-7527
- **CHENG et al.** *EMBO J.*, 1994, vol. 13, 3927-3935
- *CHEMICAL ABSTRACTS*, 290304-24-4
- *CHEMICAL ABSTRACTS*, 23541-50-6
- *CHEMICAL ABSTRACTS*, 20830-81-3
- *CHEMICAL ABSTRACTS*, 23214-92-8
- *CHEMICAL ABSTRACTS*, 25316-40-9
- *CHEMICAL ABSTRACTS*, 21672
- *CHEMICAL ABSTRACTS*, 173441-26-4
- *CHEMICAL ABSTRACTS*, 1415246-68-2
- *CHEMICAL ABSTRACTS*, 33069-62-4
- *CHEMICAL ABSTRACTS*, 1436391-86-4
- *CHEMICAL ABSTRACTS*, 108852-90-0
- *CHEMICAL ABSTRACTS*, 202350-68-3
- *CHEMICAL ABSTRACTS*, 228266-40-8
- *CHEMICAL ABSTRACTS*, 228266-41-9
- *CHEMICAL ABSTRACTS*, 38748-32-2
- *CHEMICAL ABSTRACTS*, 66547-09-9
- *CHEMICAL ABSTRACTS*, 124689-65-2
- *CHEMICAL ABSTRACTS*, 130288-24-3
- *CHEMICAL ABSTRACTS*, 35906-51-5
- *CHEMICAL ABSTRACTS*, 64-86-8
- *CHEMICAL ABSTRACTS*, 117048-59-6
- *CHEMICAL ABSTRACTS*, 723340-57-6
- *CHEMICAL ABSTRACTS*, 73-03-0
- *CHEMICAL ABSTRACTS*, 6199-67-3
- *CHEMICAL ABSTRACTS*, 18444-66-1
- *CHEMICAL ABSTRACTS*, 19054-27-4
- *CHEMICAL ABSTRACTS*, 4449-51-8
- *CHEMICAL ABSTRACTS*, 123884-00-4
- *CHEMICAL ABSTRACTS*, 152044-54-7
- *CHEMICAL ABSTRACTS*, 21150-22-1
- *CHEMICAL ABSTRACTS*, 6805-34-1
- *CHEMICAL ABSTRACTS*, 23110-15-8
- *CHEMICAL ABSTRACTS*, 30562-34-6
- *CHEMICAL ABSTRACTS*, 108073-65-0

- *CHEMICAL ABSTRACTS*, 11029-61-1
- *CHEMICAL ABSTRACTS*, 142861-00-5
- *CHEMICAL ABSTRACTS*, 52238-35-4
- *CHEMICAL ABSTRACTS*, 83329-73-1
- *CHEMICAL ABSTRACTS*, 76958-67-3
- *CHEMICAL ABSTRACTS*, 71678-03-0
- *CHEMICAL ABSTRACTS*, 87099-50-1
- *CHEMICAL ABSTRACTS*, 219989-84-1
- *CHEMICAL ABSTRACTS*, 664993-53-7
- *CHEMICAL ABSTRACTS*, 133343-34-7
- *CHEMICAL ABSTRACTS*, 225110-59-8
- *CHEMICAL ABSTRACTS*, 822520-96-7
- *CHEMICAL ABSTRACTS*, 808750-39-2
- *CHEMICAL ABSTRACTS*, 169789-55-3
- *CHEMICAL ABSTRACTS*, 267402-71-1
- *CHEMICAL ABSTRACTS*, 105049-15-8
- *CHEMICAL ABSTRACTS*, 108212-76-6
- *CHEMICAL ABSTRACTS*, 111367-04-5
- *CHEMICAL ABSTRACTS*, 11050-94-5
- *CHEMICAL ABSTRACTS*, 26645-35-2
- *CHEMICAL ABSTRACTS*, 17466-45-4
- *CHEMICAL ABSTRACTS*, 554-62-1
- *CHEMICAL ABSTRACTS*, 2738-64-9
- *CHEMICAL ABSTRACTS*, 151519-02-7
- *CHEMICAL ABSTRACTS*, 518-28-5
- *CHEMICAL ABSTRACTS*, 200625-47-4
- *CHEMICAL ABSTRACTS*, 82508-31-4
- *CHEMICAL ABSTRACTS*, 58523-30-1
- *CHEMICAL ABSTRACTS*, 21705-02-2
- *CHEMICAL ABSTRACTS*, 130743-07-6
- *CHEMICAL ABSTRACTS*, 93908-02-2
- *CHEMICAL ABSTRACTS*, 30195-30-3
- *CHEMICAL ABSTRACTS*, 87578-99-2
- *CHEMICAL ABSTRACTS*, 2447-54-3
- *CHEMICAL ABSTRACTS*, 58944-73-3
- *CHEMICAL ABSTRACTS*, 265114-54-3
- *CHEMICAL ABSTRACTS*, 2730-71-4
- *CHEMICAL ABSTRACTS*, 1148118-92-6
- *CHEMICAL ABSTRACTS*, 1310693-92-5
- *CHEMICAL ABSTRACTS*, 151038-96-9
- *CHEMICAL ABSTRACTS*, 69866-21-3
- *CHEMICAL ABSTRACTS*, 144667-38-9
- *CHEMICAL ABSTRACTS*, 1394079-41-4
- *CHEMICAL ABSTRACTS*, 1401203-09-5
- *CHEMICAL ABSTRACTS*, 1595275-71-0
- **MERO et al.** Conjugation of Poly(Ethylene Glycol) to Proteins and Peptides: Strategies and Methods. *Methods in Molecular Biology*, 2011, vol. 751, 95-129, https://doi.org/10.1007/978-1-61779-151-2_8
- Enzymatic Approaches to New Protein Conjugates. **GRIGOLETTO et al.** Polymer-Protein Conjugates. Elsevier, 2020, 271-295
- **GRIGOLETTO A et al.** *J. Drug Target.*, 2017, vol. 25 (9-10), 856-64
- **MERO, A.** ; **CLEMENTI, C.** ; **VERONESE, F. M.** ; **PASUT, G.** Covalent Conjugation of Poly(Ethylene Glycol) to Proteins and Peptides: Strategies and Methods. *Methods in Molecular Biology*, 2011, vol. 751, 95-129, https://doi.org/10.1007/978-1-61779-151-2_8